# EUROPEAN PATENT APPLICATION

(11) **EP 2 301 519 A1**
(43) Date of publication of application: **30.03.2011**
(21) Application number: 10175556.9
(22) Date of filing: 07.09.2010
(51) Int. Cl.: A61K 8/35, A61K 8/41, A61K 8/49, A61K 8/58, A61Q 17/04

(54) **Ultraviolet absorbing composition**

(30) Priority: 07.09.2009 JP 2009206477
(71) Applicant: Fujifilm Corporation, Minato-ku Tokyo (JP)
(72) Inventor: Kitagawa, Takashi, Odawara-shi, Kanagawa (JP); Yawata, Toshihiko, Minami-Ashigara-shi, Kanagawa (JP); Takeshima, Youichirou, Odawara-shi, Kanagawa (JP); Kimura, Keizo, Odawara-shi, Kanagawa (JP)
(74) Representative: HOFFMANN EITLE

(57) **Abstract**

An ultraviolet absorbing composition for a skin and hair, which has absorption of high absorbance in both UV-A region and UV-B region, wherein an ultraviolet absorbing compounds in the ultraviolet absorbing composition, will not precipitate or turn to yellow in the process of preparing cosmetics. Moreover, the ultraviolet absorbing composition shows excellent feeling in use, and will not dye clothes easily, and may be applied to the skin and hair of a living object, and exhibits excellent capability of absorbing an ultraviolet light.

The ultraviolet absorbing composition for skin and hair, comprises the ultraviolet absorbing compound represented by formula (1) and at least one ultraviolet absorbing compound selected from the group consisting of formulae (I) to (V). 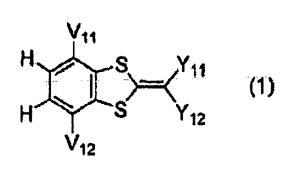 In formula (1), Y₁₁ and Y₁₂ each independently represents a monovalent substituent. One of Y₁₁ or Y₁₂ is cyano group, and the other is cyano group or an optionally substituted alkylcarbonyl group, arylcarbonyl group, heterocyclic carbonyl group, alkylsulfonyl group, arylsulfonyl group, carbamoyl group, sulfamoyl group, alkoxycarbonyl group, or aryloxycarbonyl group. V₁₁ and V₁₂ each independently represents a hydrogen atom or a monovalent substituent. The compound of formula (1) is a benzodithiol. The compounds of formulae (I) to (V) are benzophenones, a triazine, a benzotriazole and a dibenzoylmethane derivative.

## Description

### BACKGROUND OF THE INVENTION

### 1. Field of the Invention

The present invention relates to an ultraviolet (UV) absorbing composition for applying to the skin and hair of human and animals and reducing the influence due to ultraviolet light, and in particular relates to an ultraviolet absorbing composition which can effectively absorb UV-A and UV-B regions.

### 2. Description of the Related Art

It is known that human epidermis will turn to brown due to irradiattion by ultraviolet light having a wavelength in the range of 280 nm to 400 nm. Especially, it is also known that, due to light having a wavelength in the range of 280 nm to 320 nm known as UV-B light, human skin is more damaged compared with desired natural state of sun tanning, which will cause the skin to be harmfully burnt and redness to appear. Further, from the reason of health and cosmetology, there is a need for preventing the damage caused by ultraviolet ray and preventing sun tanning in skin and hair. Meanwhile, there is a need for a means that can control skin color to healthy state of sun tanning with reducing the damage caused by sun tanning. Thus, it is preferred that UV-B light is shielded.

On the other hand, it is known that UV-A light having a wavelength in the range of 320 nm to 400 nm and causing a skin to become brown may also induce the functional disorder for the skin described above. Especially, its influence is concened in a case of a sensitive skin and a skin undergoing continuous sun tanning.

UV-A light, especially, causes a skin to lose elasticity, leading to wrinkles and causes the premature aging of the skin, and it also causes skin redness to appear. In addition, this reaction can be sometimes intensified due to human constitution, causing phototoxic and photosensitive reactions to occur. Therefore, from the reason of maintaining good health or from the aesthetic reason of keeping the original skin elasticity, it is expected that the effects on skin caused by UV-A light are controlled, and UV-A light in addition to UV-B light are effectively shielded.

In order to certainly protect keratin substances in the skin and hair for ultraviolet light, an ultraviolet absorbing composition having an organic shielding agent which absorbs or shields an ultraviolet light in a range of UV-A wavelength and in a range of UV-B wavelength is generally used. However, most of the ultraviolet absorbing agents or the materials for shielding an ultraviolet light are lipophilic.

These ultraviolet absorbing agents and ultraviolet shielding agents are used in a combination of more than one or two kinds thereof, or in a combination of the organic ultraviolet absorbing agents and the ultraviolet shielding agents such as an inorganic pigment.

Moreover, they are used in various concentrations and they are used in various preparation forms for intended purposes. For example, in cosmetics suitable for skin and hair, the ultraviolet absorbing composition can take a wide variety of formats such as emulsions of oil-in-water type or water-in-oil type, gels and anhydrous products. The type and amount of the used ultraviolet absorbing composition can be appropriately selected for intended purposes. Generally, the ultraviolet absorbing agents can be mixed in the oil phase or water phase of the ultraviolet absorbing composition according to their lipophilicity and hydrophilicity.

Examples of the ultraviolet absorbing agents widely used include lipophilic 1,3,5-triazine shielding agents without containing silicon. These compounds are publicly known in cosmetic industry due to their capability of absorbing an ultraviolet light (especially UV-B light). Therefore, it is suggested to use those compounds as cosmetics composition (for example, see patent documents I to 4). The ultraviolet absorbing agents used herein can be purchased from the market, and for example, 2,4,6-tris[p-(2'-ethylhexyl-1'-oxycarbonyl)amlino]-1,3,5-triazine derivatives, available under the trade name Uvinul T150 from BASF CORP., "diethylhexyl butamido triazone" (INIC name), available under the trade name Uvasorb HEB or 2-[(p-tert-butylamide) anilino]-4,6-bis[(p-(2'-ethylhexyl-1'-oxycarbonyl)anilino-1,3,5-triazine from Sigma 3V corp., and 2,4-bis{[4,2-ethylhexyloxy]}-2-hydroxy]phenyl-6-(4-methoxyphenyl)-1,3,5-triazine derivatives, available under the trade name Tinosorb S from Ciba are especially known.

The well known ultraviolet absorbing agents which are active in the UV-B region are lipophilic compounds which are solid at normal temperature. Therefore, prescriptions and applications of the ultraviolet absorbing agents are limited when they are applied for the ultraviolet absorbing composition, especially solubilization of them is difficult and there were various kinds of the problems in selecting the solvents which are suitable and can be used for human bodies.

It is known that cinnamate derivatives such as 2-ethylhexyl 4-methoxycinnamate or isoamyl 4-methoxycinnamate are good solvents for the ultraviolet shielding agents which are difficult to be dissolved in oils, and that the cinnamate derivatives have good light protection property in UV-B range.

However, these cinnamate derivatives lack photostability, especially there are problems that they interrupts the photostability of the composition containing dibenzoylmethane derivatives.

Further, it is known that although alkyl β, β'-diphenyl acrylate or α-cyano-β, β'-diphenyl acrylate derivatives have photostability and are good solvents for the UV shielding agents which are difficult to be dissolved in oils, their ability for absorbing an ultraviolet is not sufficient in UV-B ranges.

In the case where an ultraviolet absorbing compound used in combination does not have sufficient ability for absorbing an ultrabviolet in either UV-A region or UV-B region, it is considered that the additive amount can be increased. However, there are problems that change into yellow occurs when the addtive amount is increased in order to improve light protection effect.

In addition, kinds of the ultraviolet absorbing compounds used in combination would dye clothes when contacted with the clothes or impair feeling in use when used in a skin etc. Thus, there is a need for an ultraviolet absorbing composition which can express high ultraviolet absorption property in both UV-A region and UV-B region, and good feeling in use and will not easily dye clothes etc.
[Patent Document 1] EP-A-0517104
[Patent Document 2] EP-A-0570838
[Patent Document 3] EP-A-0796851
[Patent Document 4] EP-A-0775698

### Summary of the Invention

An object of the invention in view of the problems as above is to provide an ultraviolet absorbing composition for a skin and hair, which has absorption of high absorbance in both UV-A region and UV-B region, wherein an ultraviolet absorbing compounds in the ultraviolet absorbing composition will not precipitate or turn to yellow in the process of preparing cosmetics. Moreover, the ultraviolet absorbing composition shows excellent feeling in use, and will not dye clothes easily, and may be applied to the skin and hair of a living object, and exhibits excellent capability of absorbing an ultraviolet light.

The inventors of the invention investigated and found that the above problems can be solved by making the ultraviolet absorbing composition of the invention contain an ultraviolet absorbing compound represented by the formula (1) that will described in detail below and at least one ultraviolet absorbing compound selected from a group consisting of the following formulas (I) to (V), and thereby using the compound represented by formula (1) in combination with at least one further compounds having ultraviolet absorbing ability in UV-A region or UV-B region or both regions. Then, they accomplished the invention.

That is, the consstitution of the invention is described as follows.
[1] An ultraviolet absorbing composition for skin and hair, which comprises an ultraviolet absorbing compound represented by formula (1) and at least one ultraviolet absorbing compound selected from the group of consisting of formulae (I) to (V): 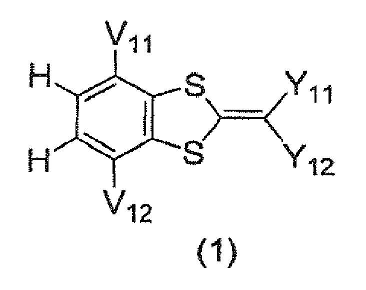 (In formula (1), Y₁₁ and Y₁₂ each independently represents a monovalent substituent; one of Y₁₁ and Y₁₂ is cyano group, and the other is cyano group, substituted or unsubstituted alkylcarbonyl group, substituted or unsubstituted arylcarbonyl group, substituted or unsubstituted heterocyclic carbonyl group, substituted or unsubstituted alkylsulfonyl group, substituted or unsubstituted arylsulfonyl group, substituted or unsubstituted carbamoyl group, substituted or unsubstituted sulfamoyl group, substituted or unsubstituted alkoxycarbonyl group, and substituted or unsubstituted aryloxycarbonyl group; and V₁₁ and V₁₂ each independently represents a hydrogen atom or a monovalent substituent.) 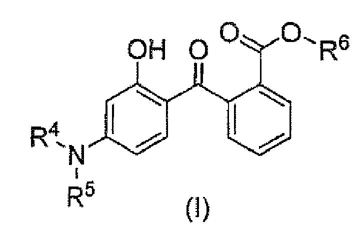 (In formula (I), R⁴ to R⁶ each independently represents a hydrogen atom, an alkyl group or a cycloalkyl group, and R⁴ and R⁵ can be linked each other to form a 5-membered ring or 6-membered ring.) 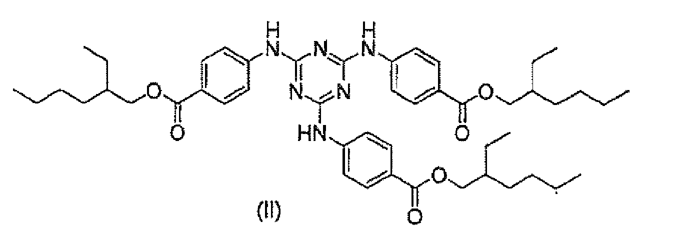 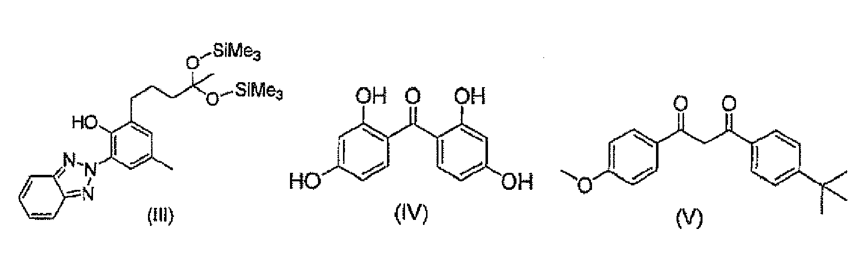
[2] The ultraviolet absorbing composition for skin and hair according to the above [1], wherein the ultraviolet absorbing compound represented by formula (1) is an ultraviolet absorbing compound represented by formula (2): 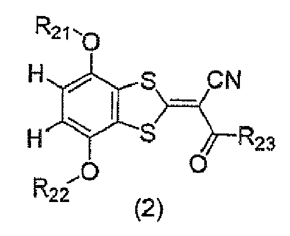 (R₂₁ and R₂₂ each independently represents unsubstituted alkyl group or unsubstituted alkylcarbonyl group. R₂₃ represents unsubstituted alkyl group or unsubstituted aryl group.)
[3] The ultraviolet absorbing composition for skin and hair according to the above [1] or [2], wherein at least one ultraviolet absorbing compound selected from the group consisting of formulae (I) to (V) is a compound represented by formula (V).

The term used herein "allowed to be used as external preparations" means that the referred compounds or materials have property that they can be applied to skin and hair, and they exhibite excellent feeling in use when they are applied to skin and hair, and they will not cause any problems in use such as unbearable discomfort such as skin and scalp irritation, allergic reaction, and unpleasant odor.

In addition, the "lipophilicity" of a compound means that it can fully be dissolved in a liquid oil phase in molecular form or it can be dissolved in a liquid oil phase in colloidal form (such as micellar form), and the mixture is uniform visually.

According to the invention, it is possible to provide an ultraviolet absorbing composition which has absorption of high absorbance in both UV-A region and UV-B region wherein an ultraviolet absorbing compounds in the ultraviolet absorbing composition will not precipitate or turn to yellow in the process of preparing cosmetics, the composition shows light stability and it becomes easy to prepare a drug formulation. The composition can be applied to skin and hair and exhibits excellent capability of absorbing an ultraviolet light.

### Brief Description of the Drawing

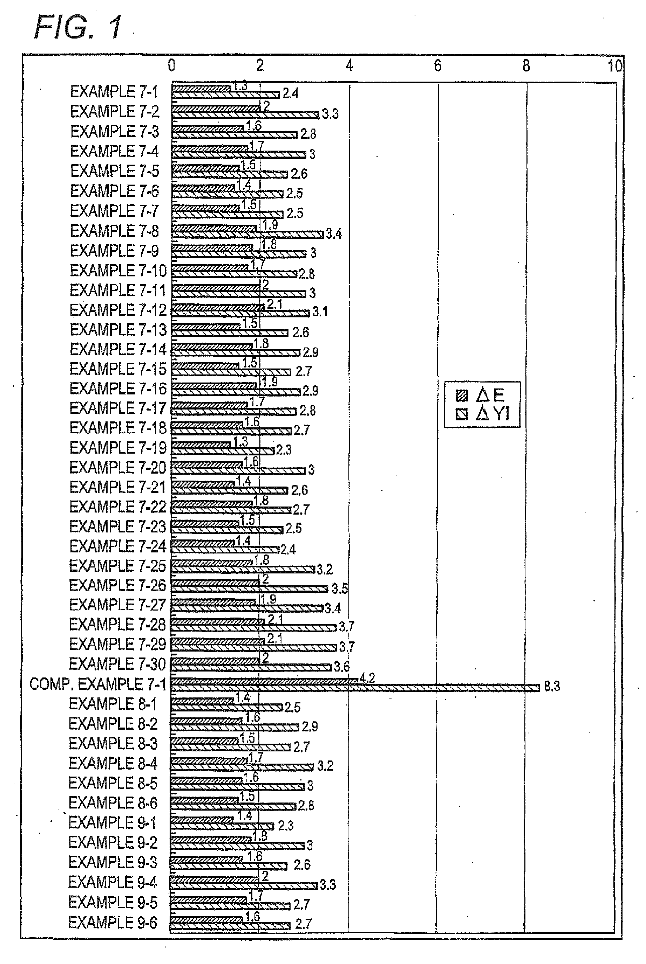

Fig. 1 shows ΔE and ΔYImeasured by a spectrophotometer.

### Detailed Description of the Invention

The invention will be described in detail below.

The ultraviolet absorbing composition of the invention for skin and hair comprises (A) an ultraviolet absorbing compound represented by the following formula (1), and (B) at least one ultraviolet absorbing compound selected from the group of consisting of the following formulae (I) to (V): 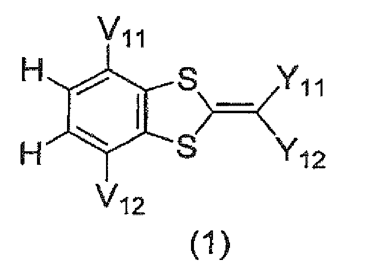 (In formula (1), Y₁₁ and Y₁₂ each independently represents a monovalent substituent, one of Y₁₁ and Y₁₂ is cyano group, and the other is cyano group, substituted or unsubstituted alkylcarbonyl group, substituted or unsubstituted arylcarbonyl group, substituted or unsubstituted heterocyclic carbonyl group, substituted or unsubstituted alkylsulfonyl group, substituted or unsubstituted arylsulfonyl group, substituted or unsubstituted carbamoyl group, substituted or unsubstituted sulfamoyl group, substituted or unsubstituted alkoxycarbonyl group, and substituted or unsubstituted aryloxycarbonyl group; and V₁₁ and V₁₂ each independently represents a hydrogen atom or a monovalent substituent.) 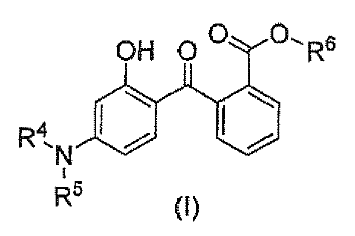 (In formula (I), R⁴ to R⁶ each independently represents a hydrogen atom, an alkyl group or a cycloalkyl group, and R⁴ and R⁵ can be linked each other to form a 5-membered ring or 6-membered ring.)

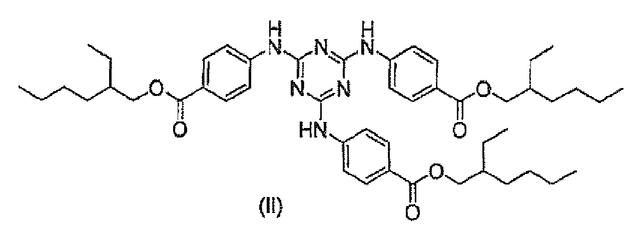

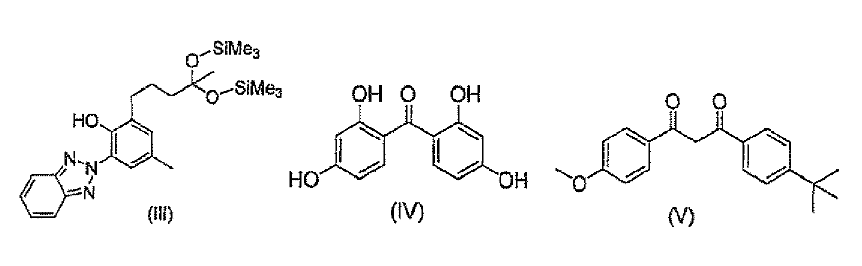

The ultraviolet absorbing composition of the invention includes an ultraviolet absorbing compound represented by formula (1) which has an absorption in UV-A region (which is called (A) ultraviolet absorbing compound hereinafter) and at least one ultraviolet absorbing compound selected from the group consisting of formulae (I) to (V) which has an absorption in UV-A region, UV-B region, and both regions (which is called (B) ultraviolet absorbing compound hereinafter). Advantages of the combination of above two compounds include: (1) the solubility of the ultraviolet absorbing composition dissolving in commonly used solvents is improved; (2) the effect of light protection in UV-A range is better; (3) turning to yellow is prevented with the increase of the additive amount. It can be said that finding the above advantages forms the basis of the invention.
(A) Ultraviolet absorbing compound represented by formula (1) (which may also be called "(A) ultraviolet absorbing compound") will be described in detail below.

### <(A) ultraviolet absorbing compound represented by the following formula (1)>

The compound (1) will be described in detail below. 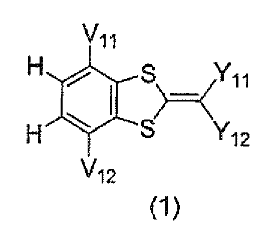 (In formula (1), Y₁₁ and Y₁₂ each independently represents a monovalent substituent; one of Y₁₁ and Y₁₂ is cyano group, and the other is cyano group, substituted or unsubstituted alkylcarbonyl group, substituted or unsubstituted arylcarbonyl group, substituted or unsubstituted heterocyclic carbonyl group, substituted or unsubstituted alkylsulfonyl group, substituted or unsubstituted arylsulfonyl group, substituted or unsubstituted carbamoyl group, substituted or unsubstituted sulfamoyl group, substituted or unsubstituted alkoxycarbonyl group, and substituted or unsubstituted aryloxycarbonyl group. V₁₁ and V₁₂ each independently represents a hydrogen atom or a monovalent substituent.)

Examples of substituted or unsubstituted alkylcarbonyl group represented by Y₁₁ and Y₁₂ include preferably an alkylcarbonyl group having a carbon number of 2 to 8, more preferably acetyl group and t-butylcarbonyl group, even more preferably t-butylcarbonyl group.

Examples of substituted or unsubstituted arylcarbonyl group represented by Y₁₁ and Y₁₂ include preferably an arylcarbonyl group having a carbon number of 2 to 14, more preferably benzoyl group and naphthoyl group, even more preferably benzoyl group.

Examples of substituted or unsubstituted heterocyclocarbonyl group represented by Y₁₁ and Y₁₂ include preferably a heterocyclocarbonyl group having a carbon number of 2-14, more preferably 2-pyridinecarbonyl group and 2-thiophenecarbonyl group, even more preferably 2-pyridinecarbonyl group.

Examples of substituted or unsubstituted alkylsulfonyl group represented by Y₁₁ and Y₁₂ include preferably alkylsulfonyl group having a carbon number of 1 to 4, for example, methanesulfonyl.

Examples of substituted or unsubstituted arylsulfonyl group represented by Y₁₁ and Y₁₂ include preferably arylsulfonyl group having a carbon number of 6 to 10, for example, benzenesulfonyl.

Examples of substituted or unsubstituted carbamoyl group represented by Y₁₁ and Y₁₂ include preferably unsubstituted carbamoyl group and an alkylcarbamoyl having a total carbon number of 1 to 9, more preferably unsubstituted carbamoyl group and an alkylcarbamoyl group having a total carbon number of 1 to 4, for example, carbamoyl, N-methylcarbamoyl, N,N-dimethylcarbamoyl and N-phenylearbamoyl.

Examples of substituted or unsubstituted sulfamoyl group represented by Y₁₁ and Y₁₂ include preferably an alkylsulfamoyl group having a total carbon number of 1 to 7, a dialkylsulfamoyl group having a total carbon number of 3 to 6, an arylsulfamoyl group having a total carbon number of 6 to 1 and a heterocyclic sulfamoyl group having a carbon number of 2 to 10, for example, sulfamoyl, methylsulfamoyl, N,N-dimethylsulfamoyl, phenylsulfamoyl and 4-pyridinesulfamoyl.

Examples of substituted or unsubstituted alkoxycarbonyl group represented by Y₁₁ and Y₁₂ include preferably an alkoxycarbonyl group having a total carbon number of 2 to 4, for example, methoxycarbonyl, ethoxycarbonyl and (t)-butoxycarbonyl, more preferably methoxycarbonyl and ethoxycarbonyl, even more preferably ethoxycarbonyl.

Examples of substituted or unsubstituted aryloxycarbonyl group represented by Y₁₁ and Y₁₂ include preferably an aryloxycarbonyl group having a carbon number of 6 to 12, more preferably an aryloxycarbonyl group having a total carbon number of 6 to 10, for example, phenyloxycarbonyl, 4-nitrophenyloxycarbonyl, 4-acetylaminophenyloxycarbonyl and 4-methanesulfonylphenyloxycarbonyl.

When monovalent substituent represented by Y₁₁ and Y₁₂ have a further substituent, as the substituent, an alkyl group, an alkoxy group and an aryl group are preferable, an alkoxy group is more preferabe.

As Y₁₁ and Y₁₂, preferably, one of Y₁₁ and Y₁₂ is cyano group, the other is cyano group, substituted or unsubstituted alkylcarbonyl group, substituted or unsubstituted arylcarbonyl group, or substituted or unsubstituted heterocyclic carbonyl group, substituted or unsubstituted carbamoyl group, and substituted or unsubstituted alkoxycarbonyl group.

More preferably, one of Y₁₁ and Y₁₂ is cyano group, and the other is cyano group, substituted or unsubstituted alkylcarbonyl group, or substituted or unsubstituted arylcarbonyl group, substituted or unsubstituted carbamoyl group, substituted or unsubstituted alkoxycarbonyl group.

Moreover, it is preferable that Y₁₁ and Y₁₂ do not form a ring with other atoms. More preferably, one of Y₁₁ and Y₁₂ is cyano group, and the other is cyano group, substituted or unsubstituted alkylcarbonyl group having a carbon number of 3 to 18, substituted or unsubstituted arylcarbonyl group having a carbon number of 7 to 18, substituted or unsubstituted carbamoyl group, substituted or unsubstituted alkoxycarbonyl group having a carbon number of 3 to 18, especially preferably cyano group, t-butylcarbonyl group, benzoyl group or ethoxycarbonyl group.

V₁₁ and V₁₂ each independently represents a hydrogen atom and a monovalent substituent. Examples of the monovalent substituent include a halogen atom, a mercapto group, cyano group, a carboxyl group, a phosphate group, sulfo group, hydroxyl group, carbamoyl group, sulfamoyl group, nitro group, alkoxy group, aryloxy group, acyl group, acyloxy group, acylamino group, sulfonyl group, sulfinyl group, sulfonylamino group, amino group, substituted amino group, an ammonium group, hydrazine group, ureido group, imide group, alkyl group or arylthio group, unsubstituted or substituted alkenylthio group, alkoxycarbonyl group, aryloxycarbonyl group, unsubstituted alkyl group, substituted alkyl group, substituted or unsubstituted aryl group. The representative examples of these substituents are the examples of Y₁₁ and Y₁₂ listed below. The substituent can be further substituted, and when there are a number of substituents, they may be same or different. The substituent in this case is the substituent described above. Moreover, the substituents can be linked each other to form a ring.

V₁₁ and V₁₂ preferably each independently represents cyano group, nitro group, hydroxyl group, alkoxy group, aryloxy group and acyloxy group, more preferably alkoxy group, aryloxy group and acyloxy group, even more preferably alkoxy group and acyloxy group.

Among them, methoxy group, ethoxy group, i-propyloxy group, 2-ethylhexyloxy group, 3,5,5-trimethylhexyloxy group, acetoxy group, propionyloxy group, n-butyryloxy group, t-butyryloxy group, 2-ethylhexanoyloxy group, 3,5,5-trimethylhexanoyloxy group are preferable, and 2-ethylhexyloxy group, 3,5,5-trimethylhexyloxy group, and 2-ethylhexanoyloxy group are more preferable.

As apreferable combination in formula (1), at least one of Y₁₁ and Y₁₂ is cyano group, and the other is substituted or unsubstituted alkylcarbonyl group having a carbon number of 3 to 18 or substituted or unsubstituted arylcarbonyl group having a carbon number of 7 to 18, and both V₁₁ and V₁₂ are alkoxy group, aryloxy group, or acyloxy group.

It is preferred that the compound represented by formula (1) is a compound represented by formula (2) or formula (3). Next, formula (2) will be described in detail below. 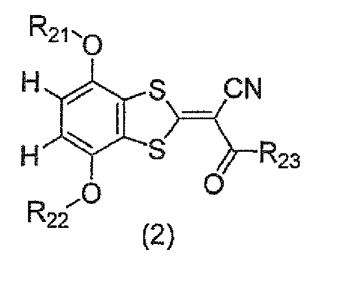 (R₂₁ and R₂₂ each independently represents unsubstituted alkyl group and unsubstituted alkykarbonyl group. R₂₃ represents unsubstituted alkyl group and unsubstituted aryl group.)

When R₂₁ and R₂₂ have a further substituent, as the substituent, hydroxyl group, an alkylcarbonyloxy group, an alkoxy group and an aryl group are preferable, hydroxyl group is more preferabe.

As the unsubstituted alkyl group represented by R₂₁ and R₂₂, unsubstituted alkyl group having a carbon number of 1 to 18 is preferable, a blanched alkyl group having a carbon number of 1 to 18 is more preferable. Specfically, methyl group, ethyl group, iso-propyl group, 2-ethylhexyl group, 3,5,5-trimethylhexyl group are more preferable, and 2-ethylhexyl group and 3,5,5-trimethylhexyl group are further more preferable.

As the unsubstituted alkylcarbonyl group represented by R₂₁ and R₂₂, unsubstituted alkylcarbonyl group having a carbon number of 1 to 18 is preferable, acetyl group, 2-ethylhexanoyl group and 3,5,5-trimethylhexanoyl group are more preferable, 2-ethylhexanoyl group and 3,5,5-trimethylhexanoyl group are even more preferable.

R₂₁ and R₂₂ each independently is preferably methyl group, 2-hydroxyethyl group, 2-ethylhexyl group, 3,5,5-trimethylhexyl group, 2-ethylhexarloyl group, or 3,5,5-trimethylhexanoyl group, and more preferably 2-ethylhexyl group, 3,5,5-trimethylhexyl group, 2-ethylhexanoyl group.

R₂₃ represents unsubstituted alkyl group having a carbon number of 2 to 18 and unsubstituted aryl group having a carbon number of 6 to 10. The unsubstituted alkyl group is preferably ethyl group, propyl group, iso-propyl group, butyl group and t-butyl group, especially preferably t-butyl group. The unsubstituted aryl group is preferably phenyl group and naphthyl group, especially preferably phenyl group. It is preferable that R₂₃ is t-butyl group or phenyl group.

As a preferable combination in formula (2), both R₂₁ and R₂₂ are 2-hydroxyethyl group, 2-ethylhexyl group, 3,5,5-trimethylhexyl group or 2-ethylhexanoyl group, and R₂₃ is t-butyl group or phenyl group.

Formula (3) will be described in detail below.

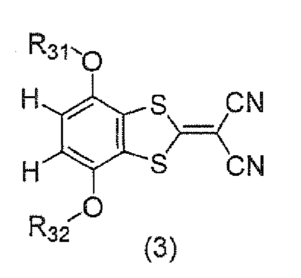

R₃₁ and R₃₂ each independently represents unsubstituted alkyl group and unsubstituted alkylcarbonyl group.

R₃₁ and R₃₂ have the same meaning as unsubstituted alkyl group and unsubstituted alkylcarbonyl group represented by R₂₁ and R₂₂ in formula (2). The preferable examples of R₃₁ and R₃₂ are same as those represented by R₂₁ and R₂₂ in formula (2).

The synthetic examples of the compound represented by formula (1) are listed below, which can be synthesized according to the synthetic methods described or cited in Journal of Chemical Crystallography, 997, 27, the 3rd line of right column on page 516 to the 15th line of right column on page 520; Liebigs Annalen der Chemie, 726, the 15th line of page 106 to the 37th line of page 109; JP 49-1115, the 7th line of left column on page 3 to the 8th line of left column on page 5; Bioorganic & Medicinal Chemistry Letters, 1997, 7, the 9th line to 19th line on page 652; Journal of Organic Chemistry, 1978,43, the 2nd line to 12th line of left column on page 2153; JP 4-338759, the 2nd line of left column on page 4 to the 2nd line of left column on page 5; JP 3-54566, the 6th line of left column on page 7 to the 10th line of left column on page 8; Synthesis, 1986, the 1st line to 22nd of left column on page 968, and so on, or according to the synthetic methods based on these methods.

Specific examples of the compound represented by formula (I) are set forth below, but the present invention is not limited thereto. In the following formulas, Et represents ethyl group and Pr represents propyl group.

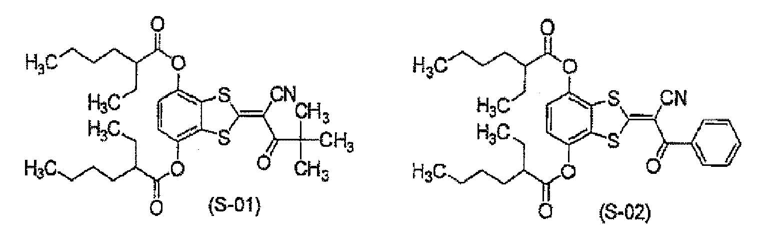

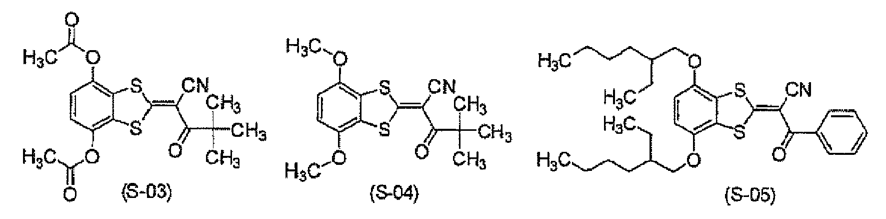

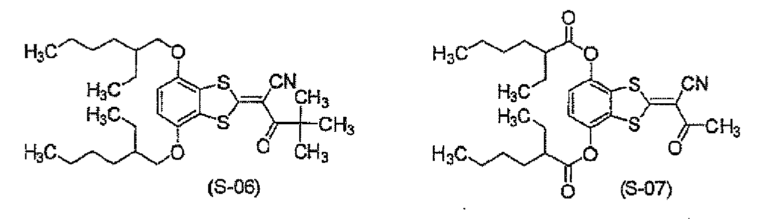

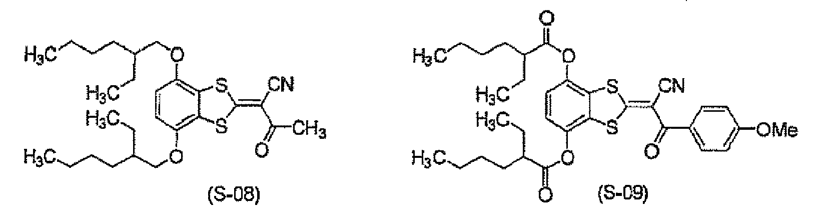

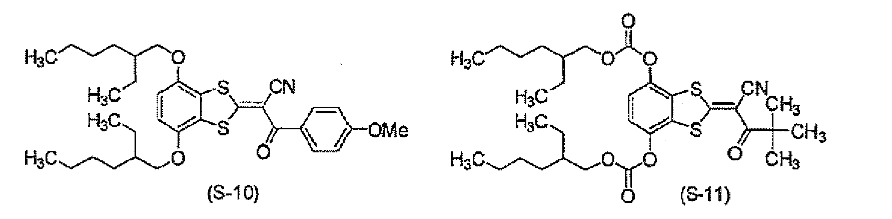

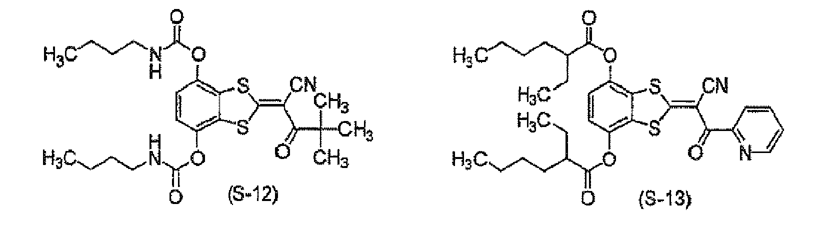

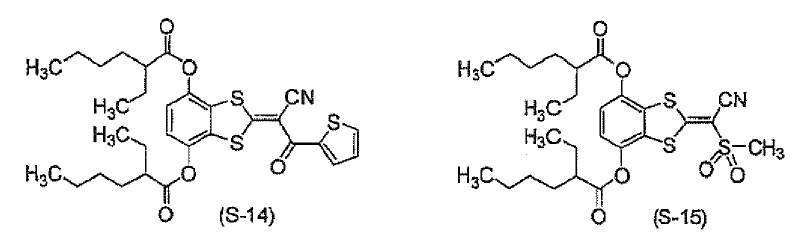

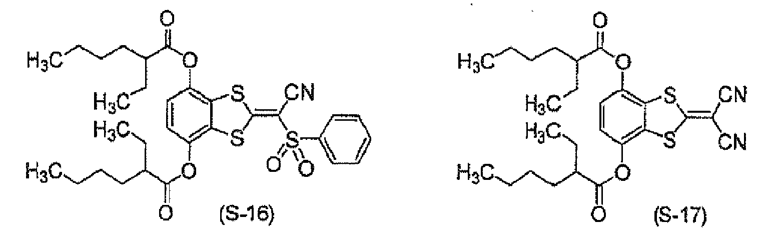

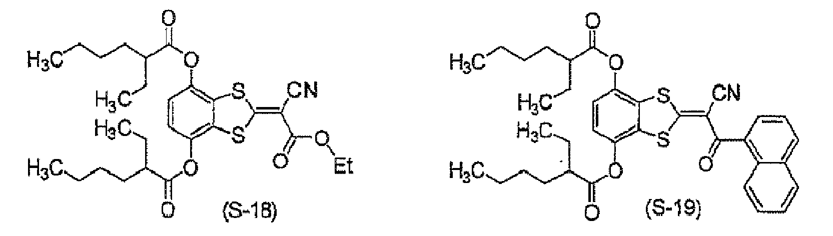

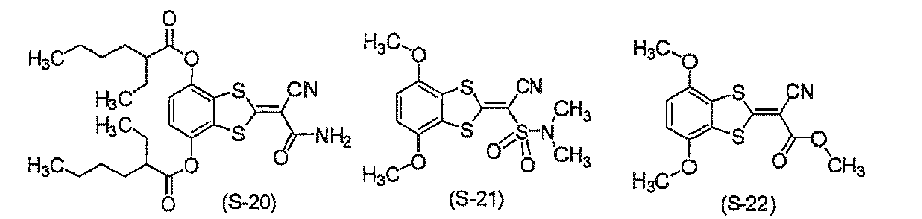

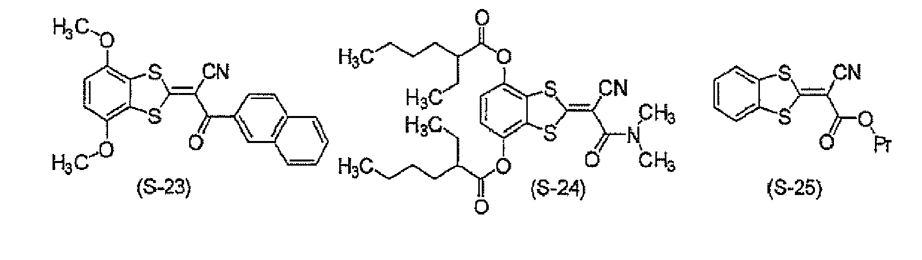

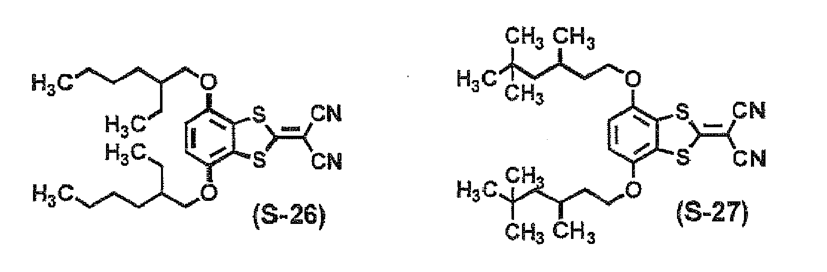

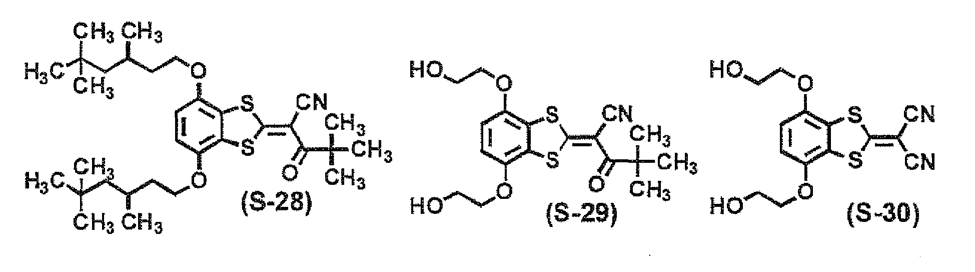

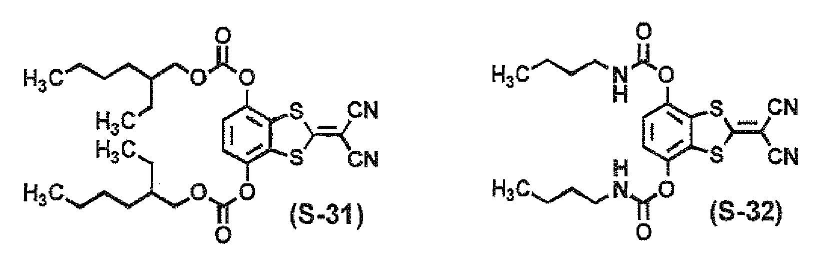

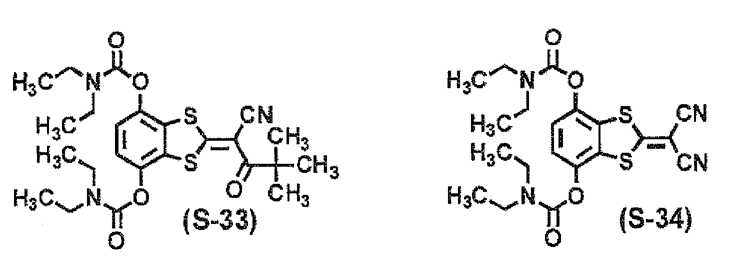

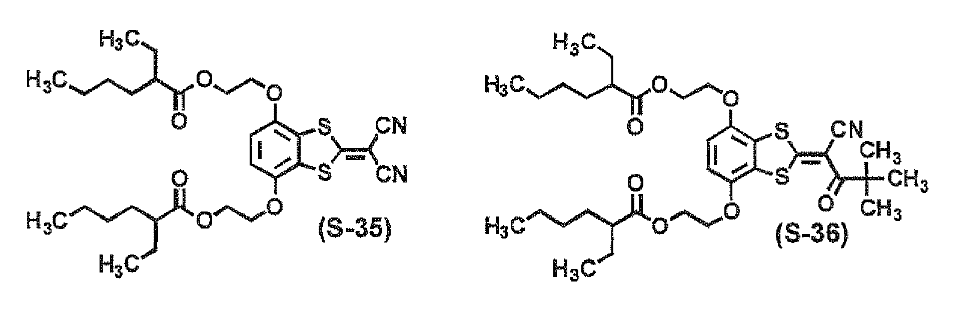

Understandably, the compounds represented by formulae (1) to (5) may contain several different tautomers depending on the structure and the surrounding environment. In the present specification, the compound is described as one representative form. The tautomer different from the description in the present specification is also contained in the compounds used in the invention.

The compounds represented by formulae (1) to (5) may contain an isotope, such as ²H, ³H, ¹³C, ¹⁵N, ¹⁷O or ¹⁸O.

The compound represented by formula (1) may be used either alone or in combination with two or more compounds. However, it is necessary that the compound is contained in an effective amount as the ultraviolet absorbing compound in the ultraviolet absorbing composition.

From the standpoint of effects, the amount of the ultraviolet absorbing compound is preferably 0.01 to 20 mass%, more preferably 0.1 to 15 mass%, based on the total solid content of the ultraviolet absorbing composition.

In a combination of the ultraviolet absorbing agents of the invention, it is preferable that the amount of the compound that has an absorption in UV-B region is large. This is because the compound that has an absorption in UV-B region has a low absorptivity. In the ultraviolet absorbing composition of the invention, the amount of (A) specific ultraviolet absorbing compound is generally 1 to 50 mass%, preferably 5 to 50 mass%, more preferably 10 to 50 mass% and more preferably 20 to 50 mass%, even more preferably 20 to 40 mass%, and most preferably 20 to 30 mass%, based on the total amout (100 mass%) of (A) ultraviolet absorbing compound and (B) ultraviolet absorbing compound.
(A) ultrabviolet absorbing compound is solid at normal temperature, which is well compatible with liquid oils (which are oily components) and fats. For example, it is well compatible with benzoate having a carbon number of 12 to 15, and easy to prepare a drug formulation.

### <(B) At least one ultraviolet absorbing compound selected from the group consisting of formulae (I) to (V)>

The ultraviolet absorbing composition of the invention comprises (B) at least one ultraviolet absorbing compound selected from the group consisting of formulae (I) to (V) (hereinafter called (B) ultraviolet absorbing compound). (B) ultraviolet absorbing compound has a different structure from (A) ultraviolet absorbing compound described above.
(B) ultraviolet absorbing compound can be suitably selected depending on the intended use of the ultraviolet absorbing composition, to the extent that the compound is capable of effectively absorbing ultraviolet light.

From a standpoint of stability and safety, (B) it is preferable that at least one selected from the group consisting of the following formulae (I) to (V) is a compound represented by formula (V).

### [Compound represented by formula(I)]

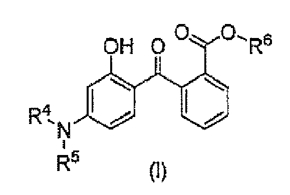

(In formula(I), R⁴ to R⁶ each individually represents a hydrogen atom, an alkyl group, and a cydoalkyl group. R⁴ and R⁵ may be linked each other to form a 5-membered or 6-membered ring.)

The alkyl group represented by R⁴ and R⁵ may be branched or linear and is substituted or unsubstituted alkyl group, preferably an alkyl group having a carbon atom of 1 to 12, methyl group, ethyl group and iso-propyl group are exemplified, and methyl group and ethyl group are preferable, and ethyl group is more preferabe.

The cycloalkyl group represented by R⁴, R⁵ and R⁶ is substituted or unsubstituted cycloalkyl group, preferably a cycloalkyl group having a carbon aton of 3 to 10, for example, cyclopentyl group and cyclohexyl group.

The alkyl group represented by R⁶ may be branched or linear and is substituted or unsubstituted alkyl group, is preferably an alkyl group having a carbon atom of 1 to 12, and hexyl group, 2-ethylhexyl group and t-butyl group are exemplefied, and hexyl group and 2-ethylhexyl group are preferable, hexyl group is more preferabe.

Such compounds are described in detail in published European patent application (EP-A) No. 1046391.

Specific examples of the compound represented by formula (I) include the following compounds.

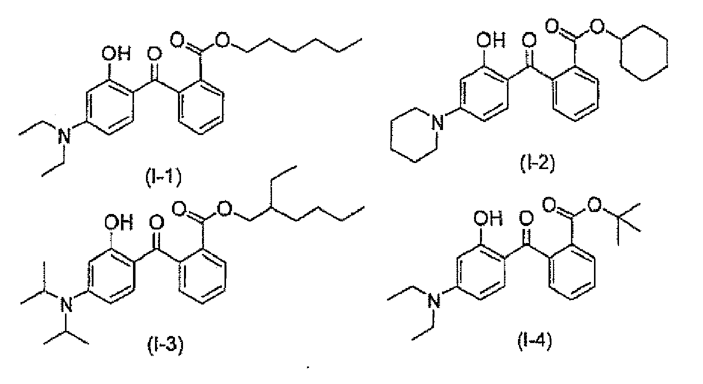

The compound represedted by the formula (1-1) is a compound publicly known as CAS No. 302776-68-7 and is commercially available under the trade name Uvinul (registered trademark) A Plus (BASF).

The compound represented by the formula (II) is a compound publicly known as CAS No. 88122-99-0 and is commercially available under the trade name Uvinul (registered trademark) T150 (BASF).

The compound represented by the formula (III) has CAS No. of 155633-54-8 and is commercially available under the trade name Mexoryl (registered trademark) XL (CHIMEX).

The compound represented by the formula (IV) has CAS No. of 131-55-5 and is commercially available under the trade name Uvinul (registered trademark) D50 (BASF).

The compound represented by the formula (V) has CAS No. of 70356-09-1 and is commercially available under the trade name Parsol (registered trademark) 1789 (Roche Vitamins).

As (B) ultraviolet absorbing compound of the invention, the compound represented by formulae (I) to (V) may be used individually or as a combination thereof. It is preferable that two kinds of the compounds represented by formulae (1) to (V) are used in combination.

Moreover, different compounds belonging to the formula (1) can be used in corobination.

The amount of (B) ultraviolet absorbing compound is preferably 0.1 to 20 mass%, more preferably 0.1 to 15 mass%, based on the total solid content of the ultraviolet absorbing composition.

When (B) ultraviolet absorbing compound is applied to the ultraviolet absorbing composition, the amount of (B) ultraviolet absorbing compound may be adjusted depending on the property of the used compound.

The ultraviolet absorbing composition of the invention can be applied to skin cosmetics, hair cosmetics, and medicinal preparations etc. Essentially, the composition includes the ultraviolet absorbing compound represented by formula (1) that has absorption in UV-A region and (B) ultraviolet absorbing compound that has absorption in UV-A region, UV-B region and both UV-A and UV-B regions. The amount of the ultraviolet absorbing agents is 0.02 to 40 mass%, preferably 0.5 to 30 mass%, more preferably 0.5 to 25 mass%, even more preferably 1 to 20 mass%, especially preferably 1 to 15 mass%, based on the total solid content of the ultraviolet absorbing composition.

### <Other Components>

The ultraviolet absorbing composition of the invention includes the specific ultraviolet absorbing compound described above and (B) ultraviolet absorbing compound.

In addition, it may also include various compounds and various materials for constituting the ultraviolet absorbing composition wherein they are allowed to be used as external preparations. The ultraviolet absorbing composition of the invention contains various preparations capable of protecting human or animal skin and hair from an ultraviolet light, for example, skin cosmetic compositions, hair cosmetic compositions, or topical medical preparations used for the skin or hair.

The ultraviolet absorbing composition of the invention includes at least two of the ultraviolet absorbing compounds described above. As long as the ultraviolet absorbing composition is a formulation which is excellent in usability and safety and can be applied to skin and hair, the other components thereof are not particularly limited, and the components can be adjusted according to the well known technique by one skilled in the art and depending to the intended purposes.

Other components which may be used for the ultraviolet absorbing composition include fats and oils or waxes, hydrocarbon oils, fatty acids, alcohols, esters, silicones and powers, all of which can be used as substrates of the ultraviolet absorbing composition. Other components also include sugars, animal and plant extracts, amino acids and vitamins, which can be used as the active components of the ultraviolet absorbing composition. Further, surfactants and color materials that can be used to adjust the substrate properties, improve appearance and feelings are also included. The above components can be selected according to intended purposes.

Organic or inorganic ultraviolet absorbing compounds which are not included in the compounds represented by formulae (I) to (V) which are exemplified as the preferable examples of the specific ulteraviolet absorbing compound and (B) ultraviolet absorbing compound can be also included. Moreover, the ultraviolet absorbing composition can include fungicides, antiseptics, antioxidants, sequestrants, perfumes and colorants, which can be included in the ultraviolet absorbing composition and other additives used as the active components in cosmetics can also be used.

According to the application of the invention, the ultraviolet absorbing composition can impart excellent ultraviolet absorption for the ultraviolet absorbing composition with various formulations such as oily, liquid, solid or powered. It can effectively shield an influence from harmful ultraviolet light when used for skin and hair. One skilled in the art, considering so as not to interfere with the effects of the ultraviolet absorbing composition of the invention, can properly select the components and the reasonable amount of the ultraviolet absorbing composition.

Next, the various components used in the ultraviolet absorbing composition will be described accordingly.

### (Fats and Oils)

Fats and oils can be used as cosmetic oil bases, cream oil phase components, emollient agents, makeup cosmetic powder binders, and grease agents and feel improving agents of shampoos and rinses.

As the fats and oils which can be used in the invention, one which includes triglycerides, i.e. triesters of fatty acids and glycerins as a main component is exemplefied. Generally, the fats and oils are different from waxes which are esters of higher fatty acids and higher alcohols.

Fats and oils are widespread in natural animals and plants, and their first application is edible. Fats and oils can also be used in various industries. Depending to an aspect at normal temperature, liquid fats and oils are called fatty oils, while solid fats and oils are called fats.

### (Oils)

Oils include mineral oils (liquid paraffin); vegetable oils (for example, sweet almond oil, macadamia oil, blackcurrant seed oil, jojoba oil, olive oil, castor oil and sunflower seed oil); synthetic oils (for example, perhydrosqualene), fatty alcohols, fatty acids, or fatty esters (for example, C12-C15 alkyl benzonate available under the trade name Witconol TN or Finsolv TN from Witco, octyl palmitate, isopropyl lanolate, or triglycerides, including those of capric/caprylic acids, or dicaprylyl carbonate available under the trade name Cetiol CC from Cognis, or oxyethylenated or oxypropylenated fatty esters and ethers); silicone oils (cyclomethicone, polydimethylsiloxane or PDMS); fluorinated oils; or polyalkylenes.

### (Waxes)

Waxes are those which include esters of higher fatty acids and higher alcohols (wax ester) as a main component and, for example, beeswaxes, lanolin, carnauba waxes, candelila waxes, and jojoba oils are exemplefied. Regardless of the components, some materials are called as waxes according to their common usages and physical forms. The waxes used for cosmetics are mainly natural wax esters.

Moreover, as wax-like compounds, hydrogenated castor oils, polyethylene waxes, and polymethylene waxes, such as Cirebelle303, available from Sasol can be exemplefied.

Hydrocarbons are generic name of compounds of carbons and hydrogens, which are classified into chain hydrocarbons and cyclic hydrocarbons according to the arrangement of carbons. The chain hydrocarbons are widely used in cosmetic raw materials. Hydrocarbons are classified into petroleum, natural minerals, synthetics, animals and plants.

The Examples of hydrocarbon oils include mineral oils (light or heavy), petrolatum (yellow or white), microcrystalline waxes, paraffin compounds or isoparaffin compounds, hydrogenated isoparaffin molecules, such as polydecene and polybutene, hydrogenated polyisobutene, squalane, isohexadecane, isododecane, and other materials derived from plants and animals.

Fatty acids can be classified into natural fatty acids and synthetic fatty acids. Natural fatty acids can be made from the fats and oils of animals and plants. Synthetic fatty acids are primarily liquid, and branched fatty acids are mostly prepared for cosmetics.

Specific examples thereof include lauric acids, myristic acids, palmitic acids, stearic acids, oleic acids, isostearic acids, etc.

Alcohols are compounds represented by formula R-OH. Alcohols with one, two, or three hydroxyl groups are called monovalent alcohols, divalent alcohols (glycols) and trivalent alcohols respectively. The aromatic hydrocarbons with their side chains replaced by a hydroxyl group are called aromatic alcohols. Generally, a higher alcohol having a carbon number of about 8 to about 24 is often used as cosmetic bases, surfactant materials and oily ingredients.

Examples of the alcohols used in cosmetics include cetyl alcohols, stearyl alcohols, cetearyl alcohols, oleyl alcohols, octyl dodecanols, for example, Guerbet alcohols based on aliphatic alcohols having a carbon number of 6 to 18, preferably 8 to 10, benzoates of alcohol having a carbon number of 12 to 15, and acetylated lanolin alcohols.

Esters are compounds obtained by a dehydration reaction of fatty acids and alcohols.

Examples of the ester oils include isopropyl myristate, isopropyl palmitate, isopropyl stearate, isopropyl isostearate, isopropyl oleate, n-butyl stearate, n-hexyl laurate, n-decyl oleate, isooctyl stearate, isononyl stearate, isononyl isononanoate, 2-ethylhexyl palmitate, 2-hexyl laurate, 2-hexyldecyl stearate, 2-octyldodecyl palmitate, oleyl oleate, oleyl erucate, erucyl oleate, erucyl erucate, cetearyl octanoate, cetyl palmitate, cetyl stearate, cetyl oleate, cetyl behenate, cetyl acetate, myristyl myristate, myristyl behenate, myristyl oleate, myristyl stearate, myristyl palmitate, myristyl lactate, propylene glycol dicaprylate/propylene glycol caprate, stearyl heptanoate, diisostearyl malate, octyl hydroxystearate.

Silicone oils and fluorine oils; Silicone oils are synthetic macromolecules wherein silicon with an organic group and oxygen are alternately linked by a chemical bond.
Silicone oils can be used for cosmetics because of their stability.

Silicones (dimethyl polysiloxanes) and organic substituted polysiloxanes and the like can be used. Specific examples thereof include dimethylpolysiloxane, methylphenylpolysiloxane, and cyclic silicone. In addition, amino-, fatty acid-, alcohol-, polyetler-, epoxy-, fluorine-, glycoside and/or alkyl-modified silicone compounds can also be properly used. These materials may be liquid at room temperature and may be resin forms and they can be used according to their preparation forms.

Examples of generalized silicones include liner polysiloxanes, dimethicones (for example, 200 oil available from Dow Coring), cyclic silicone oils, cyclopentasiloxane volatiles (for example, 345 oil available from Dow Coring), phenyltrimethcones (for example, 556 oil available form Dow Corning). Also, simethicones can be used, which are the mixtures of dimethicones having an average chain length of 200 to 300 dimethylsiloxane units and hydrogenated silicates.

Examples of fluorine oils or perfluorine oils include perflurohexane, dimethylcyclohexane, ethylcyclopentane, and polyperfloromethylisopropyl ether.

In order to adjust the viscosity or to improve the moisture of the ultraviolet absorbing composition, polyvalent alcohols can be used in the ultraviolet absorbing composition. Polyvalent alcohols are compounds having two or more hydroxyl groups in their molecules, which are called divalent alcohols or trivalent alcohols according to the number of hydroxyl groups. Polyvalent alcohols can be used in cosmetics, especially skin care cosmetics. They can be also used as a humectant for shampoos and rinses and the like.

Examples of polyvalent alcohols include ethylene glycol, diethylene glycol, triethylene glycol, polyethylene glycol, propylene glycol, glycerin, polyglycerin, 3-methyl-1,3-butanediol and 1,3-butylene glycol.

As for sugars, sugars with physiology activity and pharmacological activity and the derivatives thereof are developed and used as drugs. In application to cosmetics, sugars are often used as a humectant of skin care and hair care cosmetics, and as a softener for skin.

Examples of sugars include sorbitol, D-sorbitol, glucitol, mannitol, glucose, sucrose, lactose, maltose, maltitol and trehalose.

In the ultraviolet absorbing composition, various polymers can be used as substrates or viscosity regulators. The polymers can be called tackifiers, emulsifiers, humectants, and film-forming agents according to their functions in the ultraviolet absorbing composition.

For example, as hydrophilic tackifiers, carboxyvinyl polymers (for example, carbopol (carbomer)) and pemulen (polymer of acrylate and alkylacrylate having a carbon number of 10 to 30); polyacrylamides [such as available under the name of Sepigel 305 (CTFA name: polyacrylamide/isoparaffin having a carbon number of 13 to 14/Laureth 7) and Simulgel 600 (CTFA name: acrylamide/sodium acryloyldimethyltaurate copolymer / isohexadecane / polysorbate 80) from Seppic, which are crosslinked copolymers]; polymers and copolymers of 2-acrylamide-2-methylpropanesulfonic acid which may be crosslinked or neutralizated [such as poly (2-acrylamide-2-methylpropanesulfonic acid (CTFA name: ammonium polyacryloyldimethyltaurate), available under the name of Hostacerin AMPS from Hoechst, and Simulgel 800 (CFTA name: sodium polyacryloyldimethyltaurate/polysorbate 80/sorbitan oleate) available from Seppic]; copolymers of hydroxyethyl acrylate and 2-acrylamide-2-methylpropanesulfonic acid [such as Simulgel NS available from Seppic and Sepinov EMT10]; cellulose derivatives (such as hydroxyethylcellulose and carboxymethylcellulose); polysaccharides (especially xanthan gum); and the mixture thereof can be examplefied.

Examples of lipophilic tackifiers include synthetic polymers, for example, poly(alkyl acrylate (alkyl having a carbon number of 10 to 30)), available under the name of Intelimer-IPA13-11 and Intelimer-IPA13-6 from Landec, products available under the name of Bentone, and modified clays, such as hectorite and derivatives thereof.

Surfactants are useful when for example an emulsified ultraviolet absorbing composition is used. The surfactants may be called emulsifiers, solubilizers, dispersants and spreading agents according to their applications.

Examples of surfactants used in the ultraviolet absorbing compound include fatty alcohol polyglycol ether sulfate, monoglyceride sulfate, mono- and/or di-alkyl sulfosuccinate, fatty acid isethionate, fatty acid sarcosinate, fatty acid tauride, fatty acid glutamate, α-olefinsulfonate, ethercarboxylic acid, alkyloligoglycoside, fatty acid glucamido, alkylamido betaines and/or condensation products of protein fatty acids.

Powders and color materials; In general, powders used in cosmetics are classified into body powders, organic color materials, inorganic color materials, pearl pigments, surface treatment powers, and composite powders.

Examples of body powders include grinding products of clay minerals (such as mica and talc), synthetic inorganic powders, organic powders, metal soaps, and synthetic polymer powders.

Examples of organic color materials include tar color pigments and natural pigments. Moreover, examples of inorganic color materials include iron oxide, gunjou and carbon black. Examples of pearl pigments include mica titanium.

Animal and plant extracts can also be used as active components. They are compounds and compositions extracted from animals and plants by an extracting method. As the functions thereof, moisturizing, preserving flexible and emollient, cell-activating, and inhibiting tyrosinase activity are mainly examplefied. Their names are selected sometimes according to their function.

Examples of animal and plant extracts include sodium hyaluronate, sodium chonroitin sulfate, chitin, chitosan, collagen, elastin, peptide, lecithin, placenta essence, hematin, bovine spleen extracts, and placenta essence.

Amino acids are carboxylic acids having an amino group. Imino acids like proline and hydroxyproline are contained in amino acids. Examples of amino acids include glycine, alanine, valine, isoleucine, arginine, soluble collagen, and casein.

Vitamins are collective term of groups of organic compounds which control animal and plant nutrition in a micro amount and function as a catalyst in order to smoothly promote physiology such as procreation and metabolism, other than proteins, lipids, saccharides and inorganic salts. Examples of vitamins include vitamin A, B1, B2, B5, B6, B12, C, D2, D3, E, K1 K2, K3, biotin, nicotinic acids, and folic acids.

Moreover, an ultraviolet absorbing compound that is different from the ultraviolet absorbing compound represented by the above formula (I) and the ultraviolet absorbing compound represented by the above formula (1) can also be added to the ultraviolet absorbing composition of the invention. To the extent that the effects of the invention are not affected, a third ultraviolet absorbing compound which is not included in these compounds can also be included as a further additive.

### (Fungicides and Preservatives)

In order to prevent microbial contamination in the ultraviolet absorbing composition and to preserve product quality and safety, fungicides and preservatives can also be used in the ultraviolet absorbing composition. Fungicides can be added in cosmetics which are used to kill the bacteria growing on the skin or decrease their quantities.

Examples of fungicides and preservatives include benzoic acid, salicylic acid, sorbic acid, p-hydroxybenzoate, benzalkonium chloride, benzethonium chloride, halocalvin, and 2,4,4-trichloro-2-hydroxylphenol. In addition, as fungicides and preservatives which are used to prevent scalp bacteria considered as contributory to generation of dandruff, trichlorocarbanilide, zinc pyrithione, chamaecyparis and phenol and the like can be exemplified.

### (Antioxidants)

Fats and oils, waxes, fatty acids, esters, surfactants, perfumes and various active components are contained in cosmetics, which will gradually autoxidate to change property when they absorb oxygen in the air. This phenomenon is called rancid. Rancid can produce unpleasant odor, cause discoloration to occur, and damage safety of the products. And peroxides produced in rancid are representative skin irritant substances, which will damage human bodies. The substance which prevents axidation or delays initiation of axidation by addition is called antioxidants or antioxidant agents.

Examples of the antioxidants include amino acids (such as glycine, histidine, tyrosine, tryptophan) and derivatives thereof, imidazoles (such as urocanic acid) and derivatives thereof, peptides (such as D, L-camosine, D-carnosine, L-camosine) and derivatives thereof (such as anserine), carotinoids, carotenes (such as β-carntene, lycopene) and derivatives thereof, chlorogenic acid and derivatives thereof, lipoic acid and derivatives thereof (such as dihydroliponic acid), aurothioglucose, propylthiouracil, and other thiols (such as thioredoxin, glutathione, cysteine, cystine, cystamine and glycosyl, N-acetyl, methyl, ethyl, propyl, amyl, butyl and lauryl, palmitoyl, oleyl, γ-linoleyl, cholesteryl, and glyceryl esters thereof) and their salts, dilaurylthiodipropionate, distearylthiodipropionate, thiodipropionic acid and derivatives thereof (esters, ethers, peptides, lipids, nucleotides, nucleosides and salts thereof) and sulfoximine compounds (such as butionine sulfoximine, homocysteine sulfoximine, butionine sulfone, penta-, hexa-, and hepta-thionine sulfoximine) in very small compatible dosages (for example, pmole to µmol/kg), also (metal) chelators (for example α-hydroxy fatty acid, palmitic acid, phytic acid, lactoferrine), α-hydroxy acids (for example, citric acid, lactic acid, malic acid), humic acid, bile acid, bile extracts, bilirubin, biliverdin, EDTA and derivatives thereof, unsaturated fatty acid and derivatives thereof (for example, γ-linolenic acid, linoleic acid, oleic acid), folic acid and derivatives thereof, ubiquinone and ubiquinol and derivatives thereof, vitamin C and derivatives thereof (for example, ascorbyl palmitate, Mg ascorbyl phosphate, ascorbyl acetate), tocophenol and derivatives thereof (for example, vitamin E acetate, tocotrienol), vitamin A and derivatives (vitamin A palmitate) and coniferyl benzoate of benzoin resin, rutinic acid and derivatives thereof, α-glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, zinc and derivatives thereof (for example, ZnO and ZnSO₄), selenium and derivatives (for example selenium methionine ), stilbene and derivatives thereof (for example stilbene oxide, trans-stilbene oxide).

In addition, 2,6-di-t-butyl-4-methylphenol, n-octadecyl-β-(3,5-di-t-butyl-4-hydroxyphenyl)propionate, 1,1,3-tris(2-methyl-4-hydroxyl-5-t-butylphenyl)butane, 1,3,5-trimethyl-2,4,6-tris(3,5-di-t-butyl-4-hydroxybenzyl)-benzene, 1,3,5-tris(3,5-di-t-butyl-4-hydroxybenzyl)isocyanurate, 1,3,5-tris [β-(3,5-di-t-butyl-4-hydroxyphenyl)propionylethyl] isocyanurate, 1,3,5-tris(2,6-dimethyl-3-liydroxyl-4-t-butylbenzyl)isocyanurate, pentaerythritol tetrakis-[β-(3,5-di-t-butyl-4-hydroxyphenyl) propionate] , tris(nonylphenyl)phosphate, distearylpentaerythritoldiphosphite, tris(2,4-di-t-butylphenyl)phosphate, tris(2-t-butyl-4-methylphenyl) phosphate, bis(2,4-di-t-butylphenyl) pentaerythritoldiphosphite, tetrakis(2,4-di-t-butylphenyl)4,4'-biphenylenediphosphite, dilauryl thiodipropionate, dimyristyl thiodipropionate, distearyl thiodipropionate, pentaerythritol tetrakis (β-lauryl thiopropionate), pentaerythritol tetrakis-(β-hexyl thiopropionate), bis(2,2,6,6-tetramethylpiperidyl)sebacate, bis(1,2,2,6,6-pentamethylpiperidyl)sebacate, condensation products of 1-hydroxyethyl-2,2,6,6-tetramethyl-4-hydroxypiperidine and succinic acid, condensation products of N,N'-di(2,2,6,6-letramethylpiperidyl)hexamethylene diamine and 4-t-octylamino-2,6-dichloro-1,3,5-triazine, tris(2,2,6,6-tetramethylpiperidyl)nitrilotriacetate, tetrakis(2,2,6,6-tetramethyl-4-piperidyl) 1,2,3,4-butane tetracarboxylate, 1,1'-(1,2-ethanodiyl)-bis(3,3,5,5-tetramethylpiperazinone), condensation products of 4-amino-2,2,6,6-tetramethylpiperidine and tetramethylol acetylene can be used. (Sequestrants)

Metal ions can promote an oxidation of cosmetic raw materials, cause odor and color change to occur and produce turbidity and precipitation in transparent cosmetics, which cause cosmetics to deteriorate. In addition, metal ions can also impede the functions of drugs, causing drugs and compounds to produce colors. In order to avoid these phenomena, sequestrants can be used.

Examples of sequestrants include edetate, sodium polyphosphate, sodium citrate, gluconic acid, and tartaric acid, which contain acid groups capable of producing salts or atom groups capable of coordinating.

### (Perfume oils)

As perfume oils that impart aroma to the ultraviolet absorbing composition, perfume oil mixtures of natural and/or synthetic aromatic substances. Examples of natural perfumes include, for example, the extracts of blossoms (lily, lavender, rose, jasmine, neroli, ylang-ylang), the extracts of stems and leaves (geranium, patchouli, petitgrain), the extracts of fruits (anise, coriander, caraway, juniper), the extracts of fruit peel (bergamot, lemon, orange), the extracts of roots (mace, angelica, celery, cardamom, costus, iris, calmus), the extracts of woods (pinewood, sandalwood, guaiac wood, cedarwood, rosewood), the extracts of herbs and grasses (tarragon, lemon grass, sage, thyme), the extracts of needles and branches (tsuga sieboldii, pine, scott pine, mountain pine), te extracts og resins and balsams (galbanum, elemi, benzoin, myrrh, olibanum, opoponax). Perfumes of animal raw materials, for example derived from civet and beaver, may also be used.

Synthetic perfumes can also be used as perfume oils. Typical synthetic aromatic substances are, for example, products of ester, ether, aldehyde, ketone, alcohol or hydrocarbon type. Examples of aromatic substance compounds of the ester type are, for example, benzyl acetate, phenoxyethyl isobutyrate, p-tert-butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Examples of ethers include, for example, benzyl ethyl ether; examples of aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of ketones include, for example, ionones, isomethylionone and methyl cedryl ketone. Examples of alcohols include, for example, anethol, citronellol, eugenol, isoeugenol, geraniol, linalool, phenylethyl alcohol and terpizaeol. Examples of the hydrocarbons mainly include terpenes and balsams.

The mixtures of a number of aromatic substances are generally used for the purpose of producing an attractive perfume and keeping an effect of perfume addition.

The ether oils which have relatively low volatility and are mainly used as aroma components are salso suitable as perfume oils.

Examples thereof are sage oil, chamomile oil, clove oil, melissa oil, cinnamon leaf oil, lime-blossom oil, juniper berry oil, vetiver oil, olibanum oil, galbanum oil, labolanum oil and lavendin oil. Examples thereof also include bergamot oil, dihydromyrcenol, lilial, lyral, citronellol, phenylethyl alcohol, hexylcinnamaldehyde, geraniol, benzyl acetone, cyclamen aldehyde, linalool, boisambrene forte, ambroxan, indole, hedione, sandelice, citrus oil, mandarin oil, orange oil, allylamyl glycolate, cyclovertal, lavendin oil, muscatel sage oil, damascone, bourbon geranium oil, cyclohexyl salicylate, vertofix coeur, iso-E-Super, Fixolide NP, evernyl, iraldein gamma, phenylacetic acid, geranyl acetate, benzyl acetate, rose oxide, romillat, irotyl and floramat.

### (Colorants)

For example, colorants are described in the paper publication of Kosmetische Faerbemittel, Farbstoffkommission der Deutschem Forschungsgemeinschaft, Verlag Chemie, Weinheim, 1984, pp 81∼106.

Examples of other additives include deforming agents (such as silicone) structurants (such as maleic acid), solubilizers (such as ethylene glycol, propylene glycol, glycerin or diethylene glycol), opacifiers (such as latex, styrene/PVP or styrene/acrylamide copolymer), complexing agents (such as EDTA, NTA, alanine diacetate or phosphoric acid), propellants (such as propane/butane mixture, N₂O, dimethyl ether, CO₂, N ₂ or air), so-called coupling agents and color developing components as oxidation dye precursors, reducing agents (such as thioglycolic acid and derivatives thereof, thiolacetic acid, cysteamine, thiomalic acid and mercapto-ethane sulfonic acid), or oxidizing agents (such as hydrogen peroxide, potassium bromate or sodium bromate), which may be present in cosmetic preparation materials.

When the ultraviolet absorbing composition is used in skin cosmetics, skin roughness inhibitors, anti-aging agents, whitening agents, hair restorers can also be used as active ingredients.

### (Anti-Dandruff Agents)

Of the fungicides described above, for example, climbazole, octopirox and zinc pyrithione can be used as anti-dandruff agents.

### (Film Forming Agents)

Examples of film forming agents which can be used in the invention include, for example, chitosan, microcrystalline chitosan, quaternized chitosan, polyvinylpyrrolidone, vinyl pyrrolidone / vinyl acetate copolymer, acrylic acid, collagen, hyaluronic acid and polymers of quatemized chitosan derivatives containing the salts thereof with high proportion, and similar compounds.

### (Hydrotropic Agents)

In order to improve flowability, hydrotropic agents can be used. Examples of hydrotropic agents include, for example, ethoxylated or non-ethoxylated monoalcohols with fewer carbon atoms, diols or polyols or ethers thereof (such as ethanol, isopropanol, 1,2-dipropanediol, propylene glycol, glycerin, ethylene glycol, ethylene glycol monoethylether, ethylene glycol monobutylether, propylene glycol monomethyl ether, propylene glycol monoethyl ether, propylene glycol monobutyl ether, diethylene glycol monomethyl ether, diethylene glycol monoethyl ether, diethylene glycol monobutyl ether and similar materials thereof).

Polyols used for this purpose are preferably compounds having a carbon number of 2 to 15 and having at least two hydroxyl groups. Furthermore, polyols can have other functional groups, especially amino groups, and/or can be modified by nitrogen. Typical examples include glycerol; alkylene glycol, such as ethylene glycol, diethylene glycol, propylene glycol, butylene glycol, hexylene glycol, and polyethylene glycol with 100 to 1000 dalton average molecule weight; industry oligoglycerol mixtures with 1.5 to 10 inherent condensation degree, such as industry diglycerol mixtures with 40 to 50 mass% diglycerol; methylol compounds, such as trimethylolethane, trimethylolpropane, trimethylolbutane, pentaerythritol and dipentaerythritol; lower alkyl-glucosides, especially alkyl-glucosides having 1 to 8 carbon atoms in the alkyl group, such as methylglucoside and butylglucoside; sugar alcohols having 5 to 12 carbon atoms, such as sorbitol or mmmitol; sugars having 5 to 12 carbon atoms, such as glucose or sucrose; amino sugars, such as glucamine; dialcoholamine, such as diethanolamine or 2-amino-1,3-propanediol.

The ultraviolet absorbing composition of the invention can be properly used in combination with the compounds described above according to applications. Typical examples of formulations are listed below, but the invention is not limited to these.

### [Aqueous Composition]

Like a lotion in cosmetics, an aqueous composition mainly includes ion-exchange water and purified water, and it can be used in combination with soluble alcohols if desired. The aqueous composition is an ultraviolet absorbing composition with low viscosity, which can be prepared by adding water-soluble or water-dispersible humectants or buffers to a water phase, adding humectants and perfumes to an alcohol phase, and then mixing the water phase and the alcohol phase together.
(A) specific UV absorbing compound is preferably added to the alcohol phase. (B) ultraviolet absorbing compound is preferably added to the water phase or alcohol phase according to the type.

### [Emulsified Composition]

An emulsified composition can be a form of milky liquid (like lotion) composition with low viscosity and of creaminess composition with high viscosity. The emulsified state can be either oil-in-water (O/W) type or water in oil (W/O) type.

In preparation of the emulsified composition, a water phase with water and moisturizing components and an oil phase with oil and oil-soluble active components are prepared in advance, then, the emulsified composition can be generally obtained by imparting shear force while heating depending on the functions of surfactants in the oil phase.

The medicinal components and the ultraviolet absorbing compounds are usually added in the oil phase.

Generally, if it is milky liquid (like lotion) composition, the composition wherein the ratio of the oil phase is, for example, about 3 to about 30 mass% or 10 to 50 mass% can be examptefied, while if it is creaminess composition, the composition wherein the ratio of the oil phase is, for example, 30 to 50 mass% or 50 to 85 mass% can be exemplified.

### [Gel Composition]

Moreover, the composition of the invention can be an embodiment of a gel composition which mainly includes either water phase components or oil phase components. Water-based gels are formed by using the gelation ability of water soluble polymers. Such gels can contain oils, if the oils are added in a small amount. The oil gel composition in which only the oil phase is cured by oily gels can be formed by using surfactants and liquid crystal structures and gelling an oil-rich emulsion.

### [Solid Composition]

A solid composition which is formed by using powders such as pigment, fats and oils, and gels as binders can also be used as an embodiment of the ultraviolet absorbing composition of the invention. In this formulation, embopdiments such as what is called powdery type containing 80 to 95 mass% of powders such as pigment and 5 to 20 mass% of oil components, or oily stick type containing 35 to 60 mass% of powders and 40 to 65 mass% of oil components can be applied.

The ultraviolet absorbing composition of the invention can be in the various formulations. By applying the composition to medical external preparations and cosmetics and the like, containing various active components, it becomes possible to further impart the function of shielding ultraviolet light in addition to effective functions which these compositions inherently have.

By applying the ultraviolet absorbing composition of the invention to skin and hair, the damage to skin and hair caused by harmful ultraviolet light can be effectively prevented by a compositive function of (A) specific ultraviolet absorbing composition and (B) ultraviolet absorbing composition. Therefore, it can be used widely.

### [Examples]

The invention will be illustrated in greater detail with reference to Examples, but it should be understood that the invention is not deemed to be limited thereto.

In Examples, the compound A is the intermediate 2 described in Journal of Chemical Crystallography, 27. 997, P 516, which is the compound listed below.

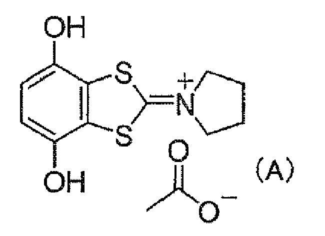

### Synthesis Example 1

### (Preparation of Illustrative Compound (S-01))

To 6.26 g of (0.02 mol) compound A were added 30 ml of N-methylpyrrolidone, and 3.00 g of (0.024 mol) pivaloylacetonitrile, and the mixture was stirred at 80°C under the nitrogen flow condition for 4 hours. After the reaction mixture was cooled, ethyl acetate and dilute hydrochloric acid were added to be treated. Then hexane was added, and after filtration, 6.10 g of the solid was obtained. 3.07 g (10 mmol) of the following compound B thus obtained was dissolved in 30 ml of tetrahydrofuran, followed by adding 1.8 g (23 mmol) pyridine, and then the mixture was cooled to 0°C. After 3.2 g (20 mmol) of 2-ethylhexanoyl chloride was added under nitrogen flow and then returned to a room temperature, the mixture was heated to 60°C and stirred for 4 hours. Then treated by ethyl acetate and dilute hydrochloric acid, after that it was isolated by silica gel column (hexane/ethyl acetate=9/1) to obtain the desired product (output: 5.3 g, yield: 47%). The maximum absorption wavelength (λ max) of Illustrative Compound (S-01) is 375 nm (EtOAc), and it was revealed that the compound has the ultraviolet absorption ability in the long wavelength region.
Mass spectrometry value: m/z 559.8
¹H NR4R (CDCl₃): δ0.90-1.00 (m, 6H), 1.02-1.11(m, 6H), 1.35-1.45(m, 8H), 1.40 (s, 9H), 1.62-1.90 (m, 8H), 2.56-2.68 (m, 2H), 7.27 (s, 2H).

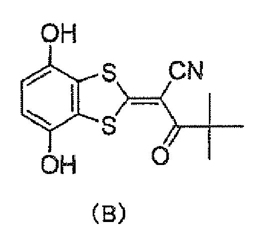

### Synthesis Example 2

### (Preparation of Illustrative Compound (S-06))

3.07 g (10 mmol) of the above B compound was dissolved in 50 ml of dimethylacetamide, followed by adding 5.5 g (24 mmol) of potassium carbonate and 4.6 g (24 mmol) of 2-ethylhexylbromide, and the mixture was stirred at 80°C for 4 hours under the nitrogen flow condition. After the mixture was treated by ethyl acetate and dilute hydrochloric acid, it was recrystallized using ethyl acetate-acetonitrile solution. The desired product was obtained (output: 8.32 g, yield: 52%). The maximum absorption wavelength (λ max) of Illustrative Compound (S-06) is 382 nm (EtOAc), and it was revealed that the compound has the ultraviolet absorption ability in the long wavelength region.
Mass spectrometry value: m/z 532.0
¹H NMR (CDCl₃) δ0.88-0.99(m, 12H), 1.28-1.39 (m, 8H), 1.42 (s, 9H), 1.43-1.56 (m, 8H), 1.71-1.80 (m, 2H), 3.90-3.99 (m, 4H), 6.80 (s, 2H)

### Synthesis Example 3

### (Preparation of Illustrative Compound (S-18))

To 6.26 g (0.02 mol) of the compound A were added 30 ml of N- methylpyrrolidone, and 2.71 g of (0.024 mol) ethyl cyanoacetate, and the mixture was stirred at 80°C under nitrogen flow for 4 hours. After the reaction mixture was cooled, ethyl acetate and dilute hydrochloric acid were added to be treated. Then hexane was added, and after filtration, 5.90 g of the solid was obtained. 2.9 g (10 mmol) of the following compound C thus obtained was dissolved in 30 ml of tetrahydrofuran, following by adding 1.8 g (23 mmol) of pyridine, and then the mixture was cooled to 0°C. After 3.1 g (20 mmol) of 2-ethylhexanoyl chloride was added under the nitrogen flow condition and then returned to room temperature, the mixture was heated to 60°C and stirred for 4 hours. Then treated by ethyl acetate and dilute hydrochloric acid, after that it was isolated by silica gel column (hexane/ethyl acetate=9/1) to obtain the desired product (output: 0.7 g, yield: 18%). The maximum absorption wavelength (λ max) of Illustrative Compound (S-18) is 60 nm (EtOAc), and it was revealed that the compound has the ultraviolet absorption ability in the long wavelength region.
Mass spectrometry value: m/z 548.7

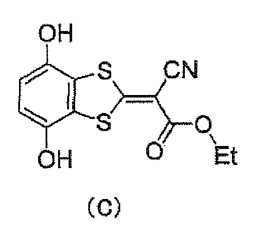

### Synthesis Example 4

### (Preparation of Illustrative Compound (S-20))

To 6.26 g (0.02 ml) of the compound A were added 30 ml of N- methylpyrrolidone, and 2.0 g (0.024 mol) of 2-cyanoacetamide, and the mixture was stirred at 70°C under nitrogen flow for 5 hours. After the reaction mixture was cooled, ethyl acetate and dilute hydrochloric acid were added to be treated. Then hexane was added, and after filtration, 4.12 g of the solid was obtained. 2.0 g (7.5 mmol) of the following compound D thus obtained was dissolved in 30 ml of tetrahydrofuran, following by adding 1.8 g (23 mmol) of pyridine, and then the mixture was cooled to 0°C. After 2.4 g (16 mmol) of 2-ethylhexanoyl chloride was added under nitrogen flow and then returned to room temperature, the mixture was heated to 60°C and stirred for 4 hours. Then treated by ethyl acetate and dilute hydrochloric acid, after that it was isolated by silica gel column (hexane/ethyl acetate=9/1) to obtain the desired product (output: 180 mg, yield: 6%). The maximum absorption wavelength (λmax) of Illustrative Compound (S-20) is 357 nm (EtOAc), and it was revealed that the compound has the ultraviolet absorption ability in the long wavelength region.
Mass spectrometry value: m/z 518.7

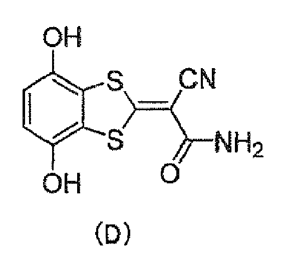

### Synthesis Example 5

### (Preparation of Illustrative Compound (S-28))

To 14.08 g (0.098 mol) of 3,5,5-trimethylhexanol, 200 ml of ethyl acetate and 11.85 g (0.117 mol) of triethylamine were added, and 12.30 g (0.107 mol) of methane sulfonyl chloride was further added while cooling at 0°C. After stirring for 2 hours, water was added, and the aqueous phase was removed to obtain the organic phase. Thereto, 10 g (0.033 mol) of the compound B, 9 g (0.065 mol) of potassium carbonate and 200 ml of dimethylacetamide were added, and the mixture was stirred at 80°C for 4 hours under the nitrogen flow condition. The resulting reaction mixture was treated with ethyl acetate and dilute hydrochloric acid and then recrystallized from an ethyl acetate-acetonitrile solution to obtain the objective product. Output: 11.83 g and yield: 65%. The maximum absorption wavelength (λmax) of Illustrative Compound (S-28) was 382 nm (EtOAc), and it was revealed that the compound has the ultraviolet absorption ability in the long wavelength region.
Mass spectrometry value: m/z 560.3
¹HNMR (CDCl₃) δ 0.91 (s, 18H), 0.99-1.01 (d, 6H), 1.11-1.30 (m, 4H), 1.4 (s, 9H), 1.61-1.86 (m, 6H), 4.03-4.H (m, 4H), 6.80 (s, 2H).

### Synthesis Example 6

### (Preparation of Illustrative Compound (S-17))

To 18.78 g (0.06 mol) of the compound A, 90 ml of N-methylpyrrolidone and 4.36 g (0.066 mol) of malonitrile were added, and the mixture was stirred at 60°C for 2 hours under the nitrogen flow condition. After cooling, the reaction mixture was treated with ethyl acetate and dilute hydrochloric acid, and 10.42 g of the solid produced by the addition of hexane was separated by filtration. Thereafter, 10 g (0.04 mol) of the obtained compound E shown below was dissolved in 100 ml of tetrahydrofuran, and 4.14 g (0.052 mol) of pyridine was added. The mixture was cooled to 0°C, and 16.38 g (0.1 mol) of 2-ethylhexanoyl chloride was added thereto. After returning to room temperature, the mixture was heated to 40°C under the nitrogen flow condition and then stirred for 1 hour. The resulting reaction mixture was treated with ethyl acetate and dilute hydrochloric acid and then recrystallized from an acetonitrile-ethyl acetate solution to obtain the objective product. Output: 16.14 g and yield: 56%. The maximum absorption wavelength (λmax) of Illustrative Compound (S-17) was 363 nm (EtOAc), and it was revealed that the compound has the ultraviolet absorption ability in the long wavelength region.
Mass spectrometry value: m/z 501.2
¹H NMR (CDClₛ) δ 0.93-0.96 (t, 6H), 1.029-1.06 (t, 6H), 1.35-1.44 (m, 8H), 1.60-1.86 (m, 8H), 2.55-2.62 (m, 2H), 7.30 (s, 2H).

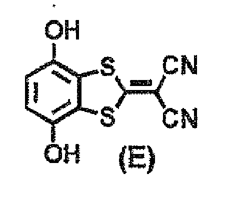

### Synthesis Example 7

### (Preparation of Illustrative Compound (S-26))

In 50 ml of dimethylacetamide, 5 g (0.02 mol) of the compound E was dissolved, and 5.57 g (0.04 mol) of potassium carbonate and 11.67 g (0.06 mol) of 2-ethylhexyl bromide were added thereto. The mixture was stirred at 70°C for 4 hours under the nitrogen flow condition, and the resulting reaction mixture was treated with ethyl acetate and dilute hydrochloric acid and then recrystallized from an ethyl acetate solution to obtain the objective product. Output: 8.20 g and yield: 86%. The maximum absorption wavelength (λmax) of Illustrative Compound (S-26) was 369 nm (EtOAc), and it was revealed that the compound has the ultraviolet absorption ability in the long wavelength region.
Mass spectrometry value: m/z 473.2 ¹H NMR (CDCl₃) δ 0.90-0.96 (m, 12H), 1.31-1.52 (m, 16H), 1.71-1.77 (m, 2H), 3.91-3.98 (m, 4H), 6.81 (s, 2H).

### Synthesis Example 8

### (Preparation of Illustrative Compound (S-27))

To 14.08 g (0.098 mol) of 3,5,5-trimethylhexanol, 200 ml of ethyl acetate and 11.85 g (0.117 mol) were added, and 12.30 g (0.107 mol) was further added while cooling at 0°C. After stirring for 2 hours, water was added, and the aqueous phase was removed to obtain the organic phase. Thereto, 8.1 g (0.033 mol) of the compound E, 9 g (0.065 mol) of potassium carbonate and 200 ml of dimethylacetamide were added, and the mixture was stirred at 80°C for 4 hours under the nitrogen flow condition. The resulting reaction mixture was treated with ethyl acetate and dilute hydrochloric acid and then recrystallized from an ethyl acetate solution to obtain the objective product. Output: 9.65 g and yield: 59%. The maximum absorption wavelength (λmax) of Illustrative Compound (S-27) was 369 nm (EtOAc), and it was revealed that the compound has the ultraviolet absorption ability in the long wavelength region.
Mass spectrometry value: m/z 501.3
¹H NMR (CDCl₃) δ 0.91 (s, 18H), 0.99-1.01 (d, 6H), 1.11-1.30 (m, 4H), 1.60-1.85 (m, 6H), 4.02-4.09 (m, 4H), 6.80 (s, 2H).

### Synthesis Example 9

### (Preparation of Illustrative Compound (S-29))

In 50 ml of dimethylacetamide, 5 g (0.016 mol) of the compound B was dissolved, and 4.05 g (0.029 mol) of potassium carbonate and 8.39 g (0.049 mmol) of 2-iodoethanol were added thereto. The mixture was stirred at 90°C for 1 hour under the nitrogen flow condition, and the resulting reaction mixture was treated with ethyl acetate and dilute hydrochloric acid and then recrystallized from an ethyl acetate solution to obtain the objective product. Output: 2.44 g and yield: 38%. The maximum absorption wavelength (λmax) of Illustrative Compound (S-29) was 382 nm (EtOAc), and it was revealed that the compound has the ultraviolet absorption ability in the long wavelength region.
Mass spectrometry value: m/z 396.1
¹H NMR ((D₃C)₂SO) δ 1.33 (s, 9H), 3.73-3.78 (m, 4H), 4.14-4.17 (m, 4H), 4.97 (s, 2H), 7.14 (s, 2H).

### Synthesis Example 10

### (Preparation of Illustrative Compound (S-30))

In 50 ml of dimethylacetamide, 4.04 g (0.016 mol) of the compound E was dissolved, and 4.05 g (0.029 mol) of potassium carbonate and 8.39 g (0.049 mmol) of 2-iodoethanol were added thereto. The mixture was stirred at 90°C for 1 hour under the nitrogen flow condition, and the resulting reaction mixture was treated with ethyl acetate and dilute hydrochloric acid and then recrystallized from an ethyl acetate solution to obtain the objective product. Output: 1.04 g and yield: 19%. The maximum absorption wavelength (λmax) of Illustrative Compound (S-30) was 369 nm (MeOH), and it was revealed that the compound has the ultraviolet absorption ability in the long wavelength region.
Mass spectrometry value: m/z 337.0
¹H NMR ((D₃C)₂SO) δ 3.71-3.74 (m, 4H), 4.13-4.16 (m, 4H), 4.97 (s, 2H), 7.19 (s, 2H).

The ultraviolet absorbing composition of the present invention is described in greater detail below by referring to Examples.

The method for preparing the formulation described in the present invention is not limited to the following preparation methods, and preparation methods for emulsions which are generally used can also be used. For example, the prescriptions described in Nikko Chemicals website can be referred to.

### [Example 1]

### Preparation of Sunscreen Cream

### [Example 1-1]

According to the prescription shown in Table 1 below, the phase (I) and the phase (II) each was heated to 70 to 80°C and uniformly dissolved. Thereafter, the phase (I) was added to the phase (II), and the mixture was stirred at 5,000 rpm for 7 minutes by means of a homomixer while keeping it at 80°C. The resulting mixture was cooled further with stirring by a paddle and after stopping the stirring at 35 to 30°C, left standing to obtain a sunscreen cream.

**Table 1**

| Phase | Components | Amount Added (mass%) |
|---|---|---|
| (I) | Emulsifier (NIKKOL Nikkomulese 41) (emulsifier produced by Nikko Chemicals) | 2.5 |
| | Behenyl alcohol (NIKKOL Behenyl Alcohol 65) (produced by Nikko Chemicals) | 2.5 |
| | Squalane (NIKKOL Squalane) (produced by Nikko Chemicals) | 4.0 |
| | Glyceryl tri-2-ethylhexanoate (NIKKOL Trifat S-308) (produced by Nikko Chemicals) | 3.0 |
| | Specific ultraviolet absorbing compound (S-01) | 2.0 |
| | Another ultraviolet absorbing compound (IV) | 2.0 |
| | Another ultraviolet absorbing compound (V) | 6.0 |
| (II) | Methyl paraben | q.s. |
| | 1,3-Butylene glycol (produced by Kyowa Hakko Chemical) | 5.0 |
| | Xanthan gum | 15.0 |
| | Purified water for the total | bal. |

### [Example 1-2 to Example 1-8]

Sunscreen creams of Example 1-2 to Example 1-8 were obtained by preparing each sunscreen cream in the same manner as in Example 1-1 except that Ultraviolet Absorbing Compound (S-06), (S-28), (S-17), (S-26), (S-27), (S-29) or (S-30) was used in place of (A) ultraviolet absorbing compound (S-01) used in the phase (I) in Example 1-1.

### [Example 2]

### Preparation of Sunscreen Cream

### [Example 2-1]

According to the prescription shown in Table 2 below, the phase (I) was previously stirred by means of a homomixer at 6,000 rpm for 10 minutes while heating at 80°C to prepare a mixture. Also, the phase (II) and the phase (III) each was heated at 80°C. The heated components of the phase (II) were added to the phase (I), and the mixture was stirred and made uniform. Thereto, the components of the phase (III) were gradually added with stirring to effect emulsification, and the emulsion obtained was stirred by means of a homomixer at 5,000 rpm for 7 minutes while keeping it at 80°C, then cooled with stirring by a paddle and after stopping the stirring at 35 to 30°C, left standing to obtain a sunscreen cream.

**Table 2**

| Phase | Components | Amount Added (mass%) |
|---|---|---|
| (I) | Condensed hexaglyceryl ricinoleate (NIKKOL Hexaglyn PR-15) (produced by Nikko Chemicals) | 1.0 |
| | Lipophilized titanium oxide fine particles | 5.0 |
| | 2-Octyldodecyl pivalate (ELEFAC 1-205) (produced by Nikko Chemicals) | 8.0 |
| | Caprylic/capric triglyceride (NIKKOL Triester F-810) (produced by Nikko Chemicals) | 3.0 |
| | Methylphenylpolysiloxane | 7.0 |
| | Decamethylcyclopentasiloxane | 2.0 |
| (II) | Emulsifier (NIKKOL Nikkomulese 41) | 2.0 |
| | Cetanol (NIKKOL Deodorant Cetanol 70) (produced by Nikko Chemicals) | 2.0 |
| | Specific ultraviolet absorbing compound (S-01) | 2.0 |
| | Another ultraviolet absorbing compound (II) | 6.0 |
| | Another ultraviolet absorbing compound (IV) | 2.0 |
| (III) | Decaglyceryl monoisostearate (NIKKOL Decaglyn 1-IS) (produced by Nikko Chemicals) | 3 |
| | Xanthan gum | 0.3 |
| | Purified water for the total | bal. |

### [Example 2-2 to Example 2-8]

Sunscreen creams of Example 2-2 to Example 2-8 were obtained by preparing each sunscreen cream in the same manner as in Example 2-1 except that Ultraviolet Absorbing Compound (S-06), (S-28), (S-17), (S-26), (S-27), (S-29) or (S-30) was used in place of (A) ultraviolet absorbing compound (S-01) used in the phase (II) in Example 2-1.

### [Example 3]

### Preparation of Hand Cream

### [Example 3-1]

According to the prescription shown in Table 3 below, the phase (I) and the phase (II) each was heated at 80°C and uniformly dissolved. Thereafter, the phase (II) was slowly added to the phase (I) with stirring by means of a homomixer, and the mixture was stirred at 5,000 rpm for 5 minutes. The resulting mixture was cooled with stirring by a paddle, and the phase (III) was added thereto at 50°C. Stirring was stopped at 35 to 30°C, and the mixture was left standing to obtain a hand cream.

**Table 3 (Hand Cream)**

| Phase | Components | Amount Added (mass%) |
|---|---|---|
| (I) | Diglyceryl monoisostearate (IS-201P) (produced by Sakamoto Yakuhin Kogyo) | 2.5 |
| | Condensed hexaglyceryl ricinoleate (NIKKOL Hexaglyn PR-15) (produced by Nikko Chemicals) | 1.0 |
| | Squalane (NIKKOL Squalane) (produced by Nikko Chemicals) | 1.0 |
| | Methylphenylpolysiloxane | 5.0 |
| | Glycerin | 10.0 |
| | Tocopherol acetate | 2.0 |
| | Methyl paraben | q.s. |
| | Specific ultraviolet absorbing compound (S-01) | 2.0 |
| | Another ultraviolet absorbing compound (I-1) | 2.0 |
| | Another ultraviolet absorbing compound (IV) | 2.0 |
| (II) | Sodium alginate | 0.1 |
| | 1,3-Butylene glycol (produced by Kyowa Hakko Chemical) | 5.0 |
| | Magnesium sulfate | 0.1 |
| | Purified water for the total | bal. |
| (III) | Liposome formulation | 5.0 |

### [Example 3-2 to Example 3-8]

Hand creams of Example 3-2 to Example 3-8 were obtained by preparing each hand cream in the same manner as in Example 3-1 except that Ultraviolet Absorbing Compound (S-06), (S-28), (S-17), (S-26), (S-27), (S-29) or (S-30) was used in place of (A) ultraviolet absorbing compound (S-01) used in the phase (I) in Example 3-1.

### [Example 4]

### Preparation of Sunscreen Gel Cream

### [Example 4-1]

According to the prescription shown in Table 4 below, the phase (I) and the phase (II) both were uniformly dissolved at room temperature, The phase (III) was heated at 70 to 80°C to dissolve uniformly and then cooled to room temperature. The phase (III) was added to the phase (I) with stirring at room temperature and when the mixture became uniform, the phase (II) was added. The resulting mixture was emulsified, uniformized and after stopping the stirring, left standing to obtain a sunscreen gel cream.

**Table 4 (Sunscreen Gel Cream)**

| Phase | Components | Amount Added (mass%) |
|---|---|---|
| (I) | Emulsifier (PEMULEN TR-1) (produced by Nikko Chemicals) | 0.2 |
| | Emulsifier (PEMULEN TR-2) (produced by Nikko Chemicals) | 0.2 |
| | Tackifier (Carbopol Ultrez 10) | 0.2 |
| | Purified water for the total | bal. |
| (II) | Triethanolamine | 0.5 |
| | Methyl paraben | q.s. |
| | Glyeerin | 5.0 |
| | Purified water for the total | 2.7 |
| (III) | Liquid paraffin (#70) | 10.0 |
| | Olive oil (NIKKOL Olive Oil) (produced by Nikko Chemicals) | 5.0 |
| | Glyceryl tri-2-ethylhexanoate (NIKKOL Trifat S-308) (produced by Nikko Chemicals) | 10.0 |
| | Specific ultraviolet absorbing compound (S-01) | 4.0 |
| | Another ultraviolet absorbing compound (III) | 5.0 |
| | Another ultraviolet absorbing compound (IV) | 5.0 |
| | dl-α-Tocopherol acetate | 0.1 |

### [Example 4-2 to Example 4-8]

Sunscreen gel creams of Example 4-2 to Example 4-8 were obtained by preparing each sunscreen gel cream in the same manner as in Example 4-1 except that Ultraviolet Absorbing Compound (S-06), (S-29), (S-17), (S-26), (S-27), (S-29) or (S-30) was used in place of (A) ultraviolet absorbing compound (S-01) used in the phase (III) in Example 4-1.

### [Example 5]

### Preparation of Hair Wax

### [Example 5-1]

According to the prescription shown in Table 5 below, the phase (I) was heated and dissolved at 85°C, and the phase (II) was heated and dissolved at 80°C. Thereafter, the phase (II) was added to the phase (I) with stirring by a paddle to effect emulsification, and the resulting emulsion was cooled to 40°C with stirring by the paddle to obtain a hair wax.

**Table 5 (Hair Wax)**

| Phase | Components | Amount Added (mass%) |
|---|---|---|
| (I) | Glyceryl stearate (NIKKOL MGS-DEX) (produced by Nikko Chemicals) | 10.0 |
| | Behenyl alcohol (NIKKOL Behenyl Alcohol 65) (produced by Nikko Chemicals) | 8.0 |
| | PEG-60 Hydrogenated castor oil (NIKKOL HCO-60) (produced by Nikko Chemicals) | 2.5 |
| | Stearic acid | 2.0 |
| | Specific ultraviolet absorbing compound (S-01) | 2.0 |
| | Another ultraviolet absorbing compound (IV) | 2.0 |
| | Another ultraviolet absorbing compound (V) | 2.0 |
| | Microcrystalline wax | 0.5 |
| | Polyethylene | 0.5 |
| | Sorbitan stearate (NIKKOL SS-10V) (produced by Nikko Chemicals) | 1.0 |
| | Mineral oil | 1.5 |
| | Hydrogenated polyisobutene | 1.5 |
| | Dimethicone | 1.0 |
| | Propyl paraben | 0.1 |
| (II) | Propylene glycol | 9.0 |
| | Methyl paraben | 0.1 |
| | Purified water for the total | bal. |

### [Example 5-2 to Example 5-8]

Sunscreen creams of Example 5-2 to Example 5-8 were obtained by preparing each hair was in the same manner as in Example 5-1 except that Ultraviolet Absorbing Compound (S-06), (S-28), (S-17), (S-26), (S-27), (S-29) or (S-30) was used in place of (A) ultraviolet absorbing compound (S-01) used in the phase (I) in Example 5-1.

### [Comparative Example 1]

The sunscreen cream of Comparative Example 1 was obtained by preparing it in the same manner as in Example 1-1 except that Tinuvin 460 produced by Ciba was used in place of (A) ultraviolet absorbing compound (S-01) used in the phase (I) in Example 1-1.

### [Comparative Example 2]

The sunscreen cream of Comparative Example 2 was obtained by preparing it in the same manner as in Example 2-1 except that Tinuvin 460 produced by Ciba was used in place of (A) ultraviolet absorbing compound (S-01) used in the phase (II) in Example 2-1.

### [Comparative Example 3]

The hand cream of Comparative Example 3 was obtained by preparing it in the same manner as in Example 3-1 except that Tinuvin 460 produced by Ciba was used in place of (A) ultraviolet absorbing compound (S-01) used in the phase (I) in Example 3-1.

### [Comparative Example 4]

The sunscreen gel cream of Comparative Example 4 was obtained by preparing it in the same manner as in Example 4-1 except that Tinuvin 460 produced by Ciba was used in place of (A) ultraviolet absorbing compound (S-01) used in the phase (III) in Example 4-1.

### [Comparative Example 5]

The hair wax of Comparative Example 5 was obtained by preparing it in the same manner as in Example 5-1 except that Tinuvin 460 produced by Ciba was used in place of (A) ultraviolet absorbing compound (S-01) used in the phase (I) in Example S-1.

### [Evaluation of Ultraviolet Absorbing Composition]

The ultraviolet absorbing compositions of Examples and Comparative Examples were evaluated as follows.

### <Transmittance Test>

Each formulation was coated to form a thin film of 10 µm, and the absorption spectrum of the film was measured. As for the absorbance, the absorption spectrum at each of 400 nm and 350 nm was measured using a UV-VIS spectrophotometer, UV-2550 (manufactured by Shimadzu Corp.), and the transmittance was determined and evaluated according to the following standards. The results are shown in Table 6 below.

### (Transmittance at 400 nm)

A: The transmittance at 400 nm was less than 5%, and the color of the composition immediately after the preparation was not yellow when observed with an eye.
B: The transmittance at 400 nm was from 5% to less than 10%, and the color of the composition immediately after the preparation was not yellow when observed with an eye.
C: The transmittance at 400 nm was 10% or more, or the color of the composition immediately after the preparation was yellow when observed with an eye.
(Transmittance at 350 nm)
A: The transmittance at 350 nm was less than 10%.
B: The transmittance at 350 nm was 10% or more.

### <Solubility>

The solubility of each of the (A) ultraviolet absorbing composition and the (B) ultraviolet absorbing composition in the oil component at the preparation of formulations was observed with an eye and evaluated according to the following standards.
A: Each compound was dissolved in the oil component immediately after blending and completely dissolved.
B: Each compound was completely dissolved in the oil component, but this required from 1 to 10 minutes.
C: Each compound was completely dissolved in the oil component. but this required 10 minutes or more.
D: Each compound was not completely dissolved in the oil component.

### [Table 6]

**Table 6**

| | Ultraviolet Absorbing Compound | | | Transmittance at 400 nm | Transmittance at 350 nm | Solubility |
|---|---|---|---|---|---|---|
| | (A) | (B) | | | | |
| Example 1-1 | (S-01) | (IV) | (V) | A | A | A |
| Example 1-2 | (S-06) | (IV) | (V) | A | A | A |
| Example 1-3 | (S-28) | (IV) | (V) | A | A | A |
| Example 1-4 | (S-17) | (IV) | (V) | A | A | A |
| Example 1-5 | (S-26) | (IV) | (V) | A | A | B |
| Example 1-6 | (S-27) | (IV) | (V) | A | A | A |
| Example 1-7 | (S-29) | (IV) | (V) | A | A | B |
| Example 1-8 | (S-30) | (IV) | (V) | A | A | B |
| Example 2-1 | (S-01) | (II) | (IV) | A | A | A |
| Example 2-2 | (S-06) | (II) | (IV) | A | A | A |
| Example 2-3 | (S-28) | (II) | (IV) | A | A | A |
| Example 2-4 | (S-17) | (II) | (IV) | A | A | A |
| Example 2-5 | (S-26) | (II) | (Itf) | A | A | B |
| Example 2-6 | (S-27) | (II) | (IV) | A | A | A |
| Example 2-7 | (S-29) | (II) | (IV) | A | A | B |
| Example 2-8 | (S-30) | (II) | (IV) | A | A | B |
| Example 3-1 | (S-01) | (I-1) | (IV) | A | A | A |
| Example 3-2 | (S-06) | (I-1) | (IV) | A | A | A |
| Example 3-3 | (S-28) | (I-1) | (IV) | A | A | A |
| Example 3-4 | (S-17) | (I-1) | (IV) | A | A | A |
| Example 3-5 | (S-26) | (I-1) | (IV) | A | A | B |
| Example 3-6 | (S-27) | (I-1) | (IV) | A | A | A |
| Example 3-7 | (S-29) | (I-1) | (IV) | A | A | B |
| Example 3-8 | (S-30) | (I-1) | (IV) | A | A | B |
| Example 4-1 | (S-01) | (III) | (IV) | A | A | A |
| Example 4-2 | (S-06) | (III) | (IV) | A | A | A |
| Example 4-3 | (S-28) | (III) | (IV) | A | A | A |
| Example 4-4 | (S-17) | (III) | (IV) | A | A | A |
| Example 4-5 | (S-26) | (III) | (IV) | A | A | B |
| Example 4-6 | (S-27) | (III) | (IV) | A | A | A |
| Example 4-7 | (S-29) | (111) | (IV) | A | A | B |
| Example 4-8 | (S-30) | (III) | (IV) | A | A | B |
| Example 5-1 | (S-01) | (V) | (IV) | A | A | A |
| Example 5-2 | (S-06) | (V) | (IV) | A | A | A |
| Example 5-3 | (S-28) | (V) | (IV) | A | A | A |
| Example 5-4 | (S-17) | (V) | (IV) | A | A | A |
| Example 5-5 | (S-26) | (V) | (IV) | A | A | B |
| Example 5-6 | (S-27) | (V) | (IV) | A | A | A |
| Example 5-7 | (S-29) | (V) | (IV) | A | A | B |
| Example 5-8 | (S-30) | (V) | (IV) | A | A | B |
| Comparative Example 1 | Tinuvin 460 produced by Ciba | (IV) | (V) | C | A | D |
| Comparative Example 2 | Tinuvin 460 produced by Gila | (II) | (IV) | C | A | C |
| Comparative Example 3 | Tinuvin 460 produced by Ciba | (I1-1) | (IV) | C | A | C |
| Comparative Example 4 | Tinuvin 460 produced by Ciba | (III) | (IV) | C | A | D |
| Comparative Example 5 | Tinuvin 460 produced by Ciba | (V) | (IV) | C | A | D |

As seen from Table 6, the ultraviolet absorbing composition of the present invention has excellent ultraviolet absorption property in both wavelength regions of 350 nm and 400 nm and is excellent in ultraviolet shielding property.

On the other hand, in both of Comparative Examples 1 and 2 where known ultraviolet absorbing compounds are used individually or in combination, the ultraviolet shielding property at 350 nm or 400 nm is insufficient.

### <Sensory Evaluation>

With respect to various formulations prepared in Example 1-1 to Example 5-8 and Comparative Examples 1 to 5, the formulation was actually applied to skin or hair by using fingers and its feeling was evaluated. The evaluation was performed by 10 monitors, as a result, out of 10 monitors, 10 persons (all monitors) judged that the formulation was smoothly spread by fingers and gave good feeling without sticking.

### [Example 6]

### Preparation of Sunscreen Cream

According to the prescription shown in Table 7 below, the fat phase (I) was heated to 70°C. the aqueous phase (II) was heated in a final beaker, and the phase (III) was prepared by dispersing powders in oils. The fat phase (I) was emulsified in the aqueous phase (II) by stirring using a rotor stator, and the phase (III) was introduced with rapid stirring. The resulting mixture was then slowly stirred until it returned to ambient temperature, and this mixture was neutralized with the phase (IV) to obtain a sunscreen cream.

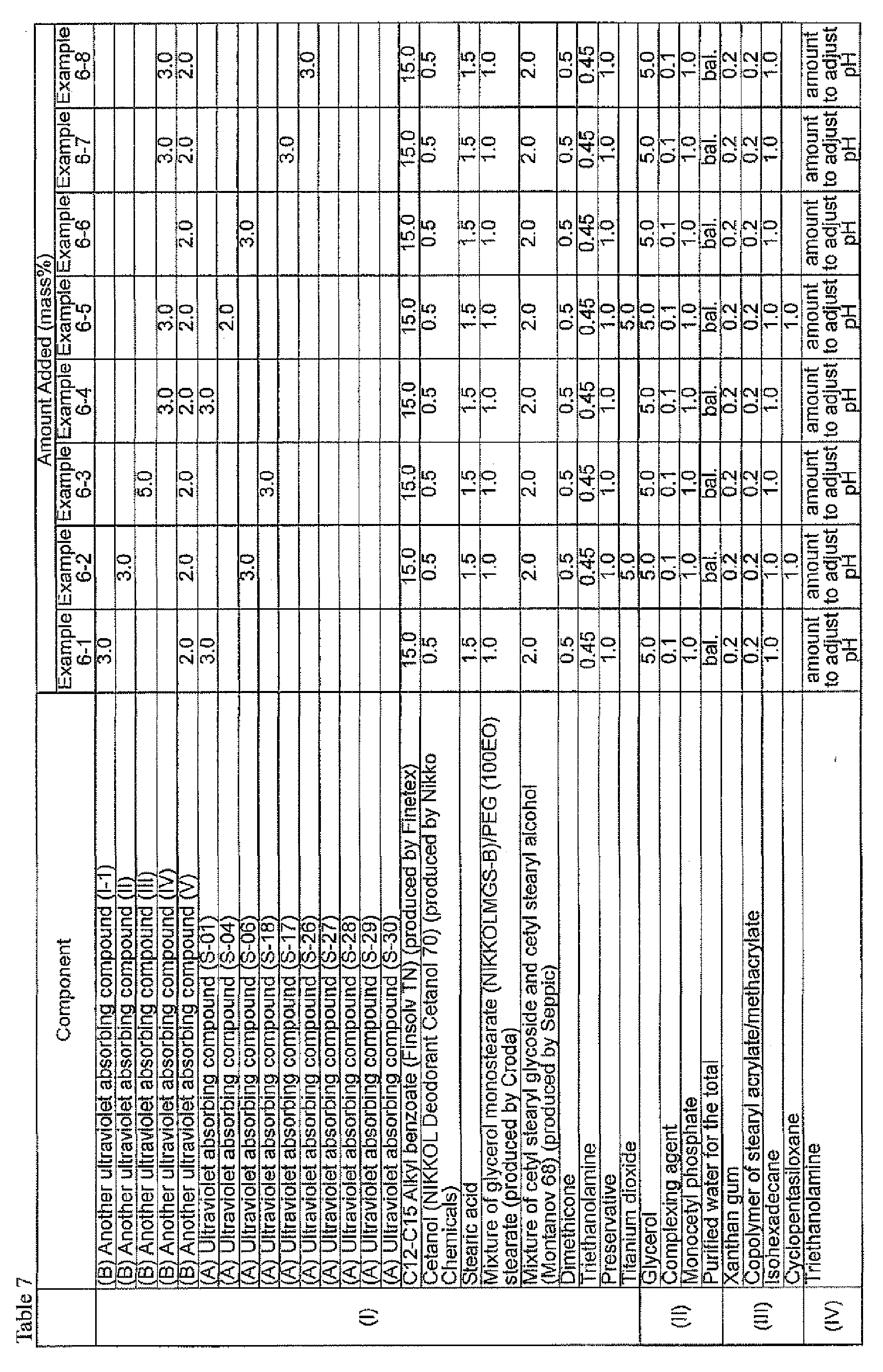

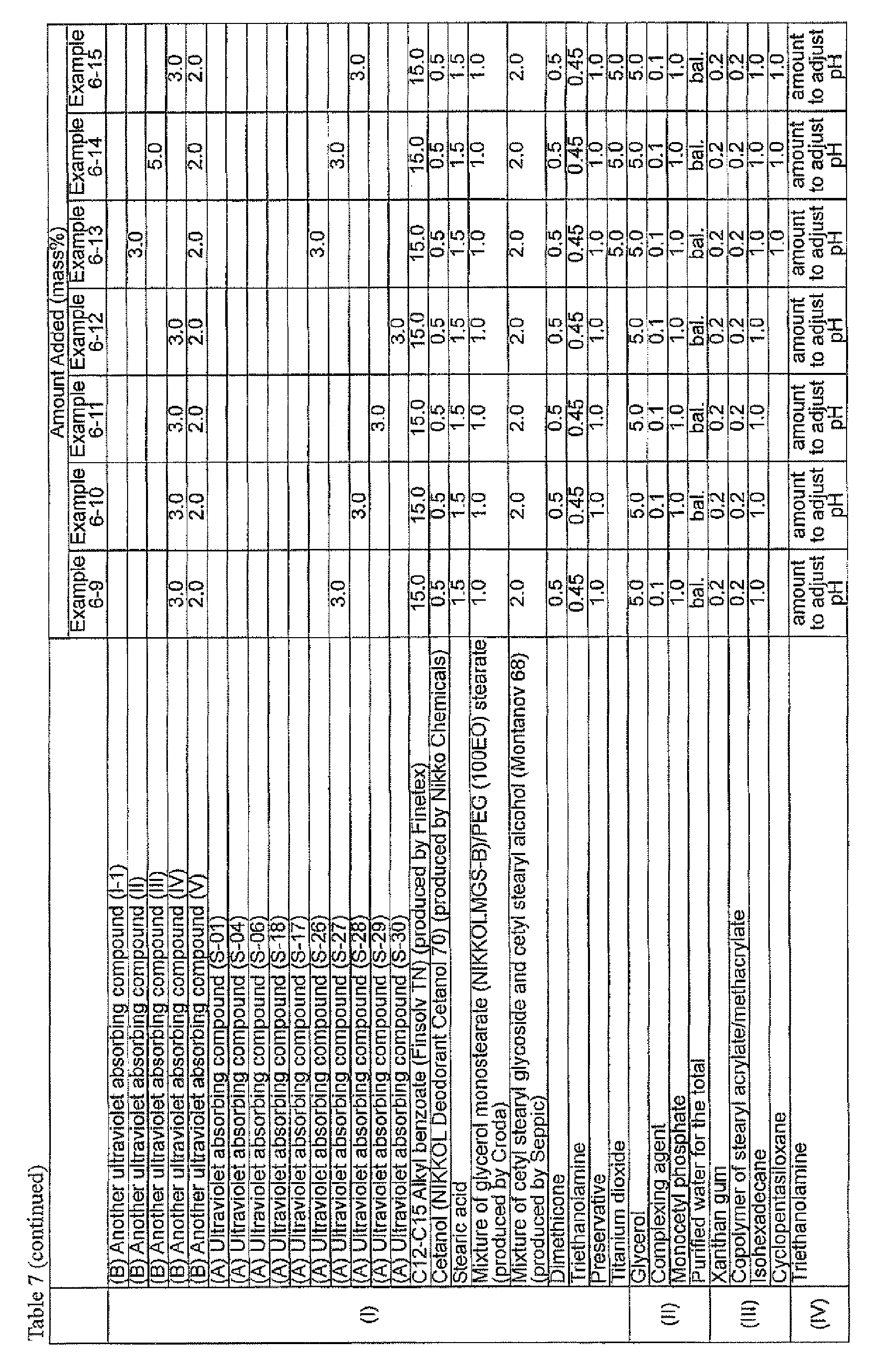

### [Comparative Example 6-1]

The sunscreen cream of Comparative Example 6-1 was obtained by preparing it in the same manner as in Example 6-1 except that Tinuvin 460 (CAS No. 208343-47-9) produced by Ciba was used in place of (A) ultraviolet absorbing compound (S-01) used in the phase (I) in Example 6-1.

### [Comparative Example 6-2]

The sunscreen cream of Comparative Example 6-2 was obtained by preparing it in the same manner as in Example 6-2 except that Tinosorb S (CAS No. 187393-00-6) produced by Ciba was used in place of (A) ultraviolet absorbing compound (S-06) used in the phase (I) in Example 6-2.

### [Comparative Example 6-3]

The sunscreen cream of Comparative Example 6-3 was obtained by preparing it in the same manner as in Example 6-3 except that NeoHeliopan-AP (CAS No. 180898-37-7) produced by Symrise was used in place of (A) ultraviolet absorbing compound (S-18) used in the phase (I) in Example 6-3.

### [Comparative Example 6-4]

The sunscreen cream of Comparative Example 6-4 was obtained by preparing it in the same manner as in Example 6-4 except that Uvinul N-539 (CAS No. 6197-30-4) produced by BASF was used in place of (A) ultraviolet absorbing compound (S-01) used in the phase (I) in Example 6-4.

### [Comparative Example 6-5]

The sunscreen cream of Comparative Example 6-5 was obtained by preparing it in the same manner as in Example 6-5 except that Uvinul N-539 (CAS No. 6197-30-4) produced by BASF was used in place of (A) ultraviolet absorbing compound (S-04) used in the phase (I) in Example 6-5.

### [Comparative Example 6-6]

The sunscreen cream of Comparative Example 6-6 was obtained by preparing it in the same manner as in Example 6-6 except that Uvinul N-539 (CAS No. 6197-30-4) produced by BASF was used in place of (A) ultraviolet absorbing compound (S-06) used in the phase (I) in Example 6-6.

### [Comparative Example 6-7]

The sunscreen cream of Comparative Example 6-7 was obtained by preparing it in the same manner as in Example 6-7 except that NeoHeliopan-AP (CAS No. 180898-37-7) produced by Symrise was used in place of (A) ultraviolet absorbing compound (S-17) used in the phase (I) in Example 6-7.

### [Comparative Example 6-8]

The sunscreen cream of Comparative Example 6-8 was obtained by preparing it in the same manner as in Example 6-10 except that Uvinul N-539 (CAS No. 6197-30-4) produced by BASF was used in place of (A) ultraviolet absorbing compound (S-28) used in the phase (I) in Example 6-10.

### [Comparative Example 6-9]

The sunscreen cream of Comparative Example 6-9 was obtained by preparing it in the same manner as in Example 6-13 except that Uvinul N-539 (CAS No. 6197-30-4) produced by BASF was used in place of (A) ultraviolet absorbing compound (S-26) used in the phase (I) in Example 6-13.

### [Comparative Example 6-10]

The sunscreen cream of Comparative Example 6-10 was obtained by preparing it in the same manner as in Example 6-14 except that NeoHeliopan-AP (CAS No. 180898-37-7) produced by Symrise was used in place of (A) ultraviolet absorbing compound (S-27) used in the phase (I) in Example 6-14.

### [Evaluation of Ultraviolet Absorbing Composition]

The ultraviolet absorbing compositions of Examples and Comparative Examples were evaluated as follows.

### <Sensory Evaluation>

With respect to various formulations prepared in Examples 6-1 to 6-15 and Comparative Examples 6-1 to 6-10, the formulation was actually applied to skin or hair by using fingers and its feeling was evaluated. The evaluation was performed by 10 monitors

As a result, with respect to Examples 6-1 to 6-15, out of 10 monitors, 10 persons (all monitors) judged that the formulation was smoothly spread by fingers and gave good feeling without sticking. With respect to Comparative Examples 6-1 to 6-10, the number of persons who judged the formulation as good is shown in Table 8.

**Table 8**

| Comparative Example | Ultraviolet Absorbing Compound | | | Spreading | Sticking |
|---|---|---|---|---|---|
| 6-1 | Tinuvin 460 produced by Ciba (CAS No. 208343-47-9). | (I-1) | (V) | 7 persons | 6 persons |
| 6-2 | Tinosorb S produced by Ciba (CAS No. 187393-00-6) | (II) | (V) | 6 persons | 8 persons |
| 6-3 | NeoHeliopan-AP produced by Symrise (CAS No. 180898-37-7) | (III) | (V) | 4 persons | 7 persons |
| 6-4 | Uvinul N-539 produced by BASF (CAS No. 6197-30-4) | (IV) | (V) | 6 persons | 7 persons |
| 6-5 | Uvinul N-539 produced by BASF (CAS No. 6197-30-4) | (IV) | (V) | 5 persons | 8 persons |
| 6-6 | Uvinul N-539 produced by BASF (CAS No. 6197-30-4) | (V) | - | 6 persons | 8 persons |
| 6-7 | NeoHeliopan-AP produced by Symrise (CAS No. 180898-37-7) | (IV) | (V) | 5 persons | 7 persons |
| 6-8 | Uvinul N-539 produced by BASF (CAS No. 6197-30-4) | (IV) | (V) | 8 persons | 8 persons |
| 6-9 | Uvinul N-539 produced by BASF (CAS No. 6197-30-4) | (II) | (V) | 6 persons | 7 persons |
| 6-10 | NeoHeliopan-AP produced by Symrise (CAS No. 180898-37-7) | (III) | (V) | 6 persons | 8 persons |

### [Example 7]

### [Metal Oxide Powder Surface-Treated with Alkylalkoxysilane]

Titanium oxide powder (average particle diameter: 0.015×0.06 µm) (2 kg) was formed into an aqueous slurry, and the pH thereof was adjusted to 3 with 20% sulfuric acid. Thereto 100 g of octyltriethoxysilane was added, and the mixture was stirred for 30 minutes and after adjusting the pH to 10 with sodium hydroxide, ripened for 1 hour. This mixture was neutralized, filtered, washed, dried and then pulverized using a jet mill to obtain titanium oxide powder surface-treated with octyltriethoxysilane. Incidentally, zinc oxide powder, iron oxide powder and cerium oxide powder each surface-treated with octyltriethoxysilane are obtained by replacing the titanium oxide powder by zinc oxide powder, iron oxide powder and cerium oxide powder, respectively.

Also, various metal oxide powders surface-treated with octyltrimethoxysilane are obtained in the same manner.

### [Examples 7-1 to 7-12, Comparative Example 7-1: Preparation of W/O Sunscreen]

The W/O sunscreens shown in Table 9 below were prepared by the conventional method, and their dyeing property by secondary adhesion was examined.

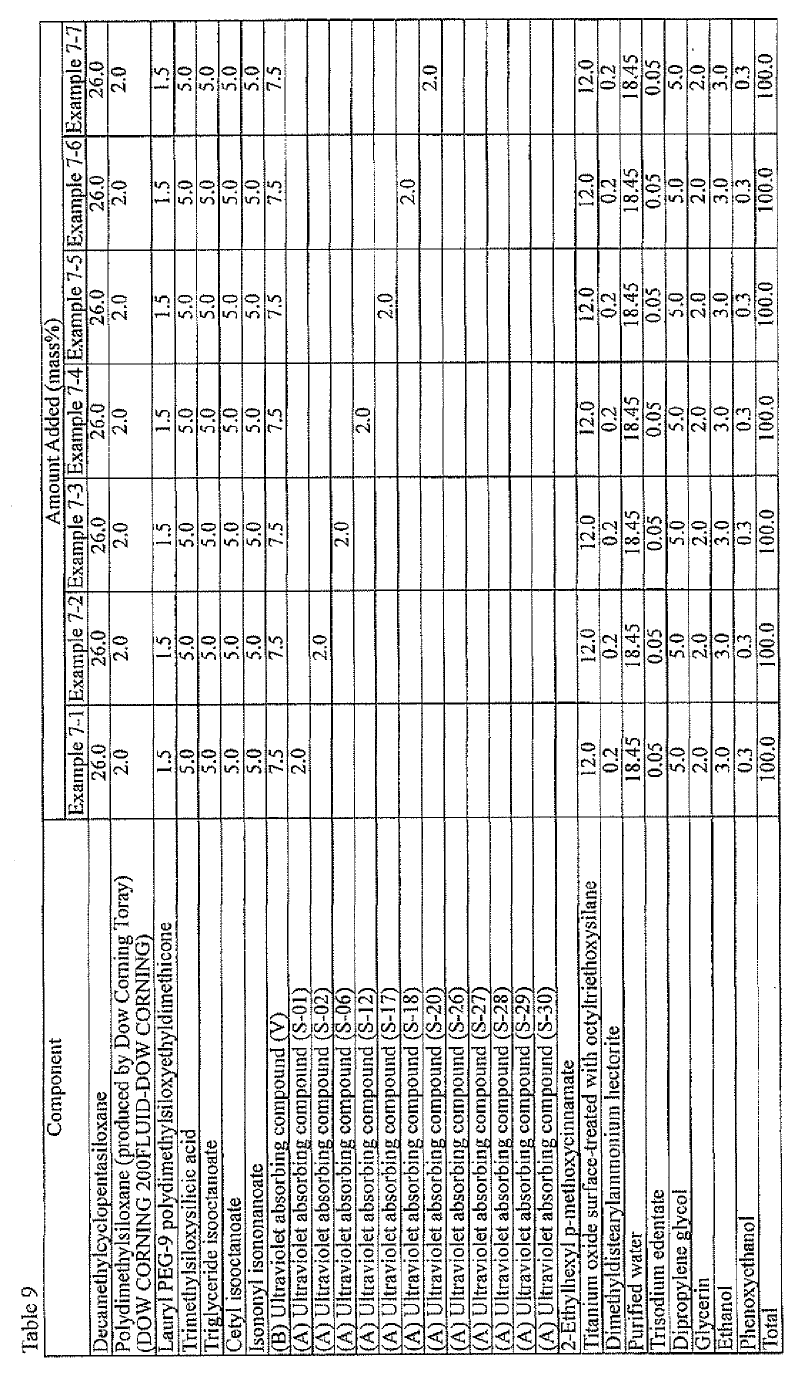

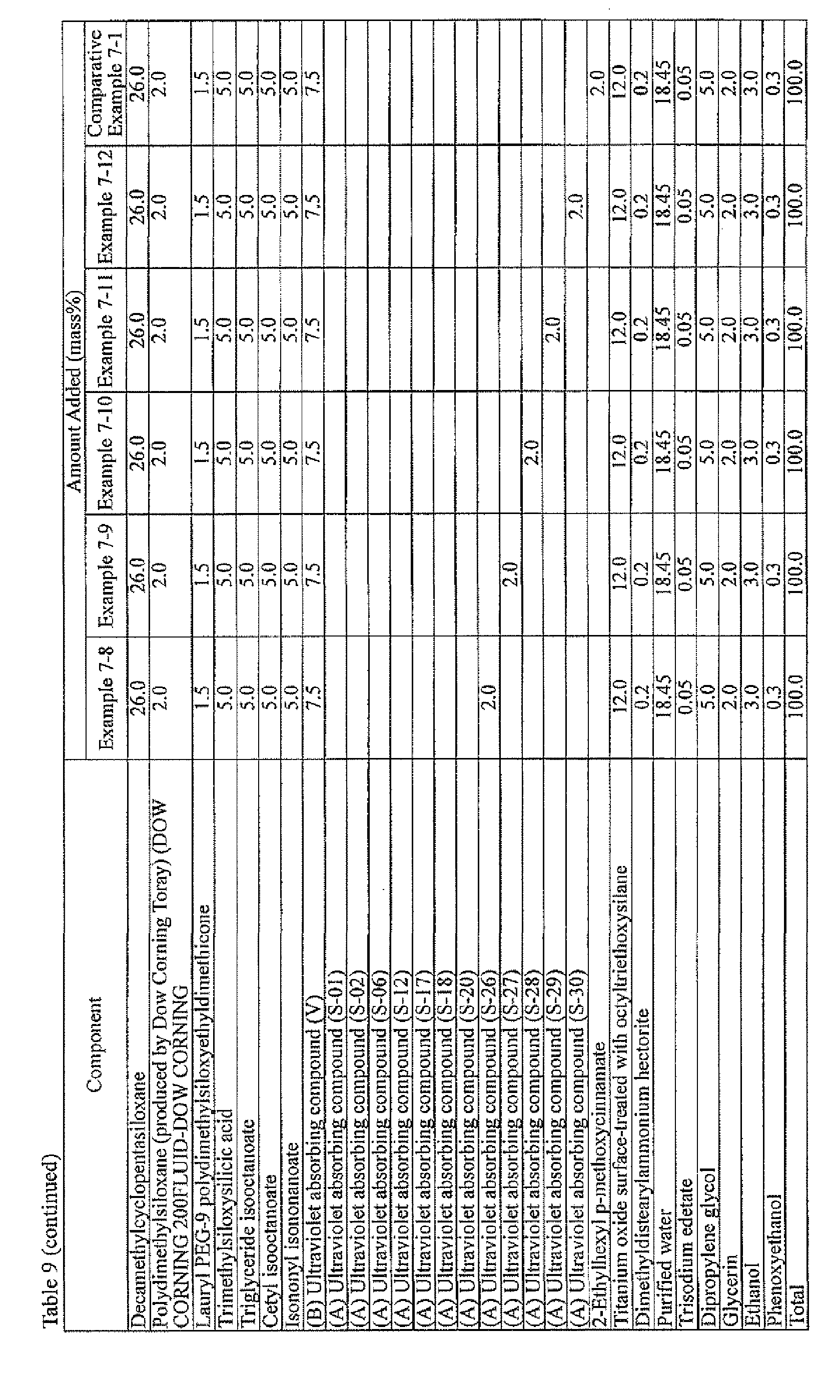

### [Measuring Method of Dyeing Property]

The measurement was performed by the method where as shown in Fig. 1 at page 12 of JP-A-2007-320917, each sample of Examples 7-1 to 7-12 and Comparative Example 7-1 was thickly applied to an arm and then transferred to the center of cotton broadcloth (amount transferred: about 0.06 g) and after left standing in a room for one day, the cloth was washed using a normal detergent for clothes and measured for ΔE and ΔYI by a spectrophotometer (CM-2002, manufactured by Minolta, called Konica Minolta Censing Inc. now). Fig. 1 shows the results.

It is seen from Fig. 1 that dyeing property in Examples is significantly reduced as compared with Comparative Example 7-1.

Prescription examples of the sunscreen cosmetic of the present invention are set forth below. All are a sunscreen cosmetic exhibiting reduced dyeing property for clothes and exerting excellent ultraviolet absorption ability in the UV-A region.

### [Examples 7-13 to 7-18: Sunscreen Cosmetic, W/O Emulsion]

**Table 10**

| Components | Amount Added (mass%) | | | | | |
|---|---|---|---|---|---|---|
| | Example 7-13 | Example 7-14 | Example 7-15 | Example 7-16 | Example 7-17 | Example 7-18 |
| Polydimethylsiloxane (produced by Dow Corning Toray) (DOW CORNING 200 FLUID-DOW CORNING) | 1.0 | 1.0 | 1.0 | 1.0 | 1.0 | 1.0 |
| Decamethylcyclopentasiloxane | 25.0 | 25.0 | 25.0 | 25.0 | 25.0 | 25.0 |
| Trimethyisiloxysilicic acid | 5.0 | 5.0 | 5.0 | 5.0 | 5.0 | 5.0 |
| Lauryl PEG-9 polydimethylsiloxyethyldimethicone | 1.0 | 1.0 | 1.0 | 1.0 | 1.0 | 1.0 |
| lsononyl isononanoate | 5.0 | 5.0 | 5.0 | 5.0 | 5.0 | 5.0 |
| (B) Ultraviolet absorbing compound (IV) | 7.5 | 7.5 | 7.5 | 7.5 | 7.5 | 7.5 |
| Butyl ethyl propanediol | 0.5 | 0.5 | 0.5 | 0.5 | 0.5 | 0.5 |
| (B) Ultraviolet absorbing compound (V) | 3.0 | 3.0 | 3.0 | 3.0 | 3.0 | 3.0 |
| (A) Ultraviolet absorbing compound (S-01) | 4.0 | | | | | |
| (A) Ultraviolet absorbing compound (S-06) | | 4.0 | | | | |
| (A) Ultraviolet absorbing compound (S-17) | | | 4.0 | | | |
| (A) Ultraviolet absorbing compound (S-26) | | | | 4.0 | | |
| (A) Ultraviolet absorbing compound (S-27) | | | | | 4.0 | |
| (A) Ultraviolet absorbing compound (S-28) | | | | | | 4.0 |
| Dimethyldistearylammonium hectorite | 0.5 | 0.5 | 0.5 | 0.5 | 0.5 | 0.5 |
| Polyalkyl acrylate spherical powder | 5.0 | 5.0 | 5.0 | 5.0 | 5.0 | 5.0 |
| Zinc oxide surface-treated with octyitriethoxysilane | 15.0 | 15.0 | 15.0 | 15.0 | 15.0 | 15.0 |
| Dipropylene glycol | 5.0 | 5.0 | 5.0 | 5.0 | 5.0 | 5.0 |
| Phenoxyethanol | 0.5 | 0.5 | 0.5 | 0.5 | 0.5 | 0.5 |
| Methanol | 2.0 | 2.0 | 2.0 | 2.0 | 2.0 | 2.0 |
| Phenylene-1,4-bis(2-benzimidazyl)-3, 3'-5,5'-tetrasulfonic acid | 2.0 | 2.0 | 2.0 | 2.0 | 2.0 | 2.0 |
| Triethanolamine | 1.0 | 1.0 | 1.0 | 1.0 | 1.0 | 1.0 |
| Purified water | bal. | bal. | bal. | bal. | bal. | bal. |

### Production Method

According to the prescription shown in Table 10 below, the aqueous phase was gradually added to the oil phase and after the completion of addition, the mixture was stirred using a stirrer ((MAZELA) Z-1000, manufactured by Tokyo Rikakikai Co., Ltd.) to make uniform the emulsified particles, whereby the emulsion was prepared.

### [Examples 7-19 to 7-24: Sunscreen Cosmetic, W/O Emulsion]

**Table 11**

| | Component | Amount Added (mass%) | | | | | |
|---|---|---|---|---|---|---|---|
| | | Example 7-19 | Example 7-20 | Example 7-21 | Example 7-22 | Example 7-23 | Example 7-24 |
| 1 | Polyoxyethylene hydrogenated castor oil | 1.0 | 1.0 | 1.0 | 1.0 | 1.0 | 1.0 |
| 2 | Dimethicone copolyol | 0.5 | 0.5 | 0.5 | 0.5 | 0.5 | 0.5 |
| 3 | Decamethylcyclopentasiloxane | 15.0 | 15.0 | 15.0 | 15.0 | 15.0 | 15.0 |
| 4 | Isostearic acid | 0.5 | 0.5 | 0.5 | 0.5 | 0.5 | 0.5 |
| 5 | Phenyl trimethicone | 1.0 | 1.0 | 1.0 | 1.0 | 1.0 | 1.0 |
| 6 | Titanium oxide surface-treated with octyltrimethoxysilane | 5.0 | 5.0 | 5.0 | 5.0 | 5.0 | 5.0 |
| 7 | (A) Ultraviolet absorbing compound (S-01) | 4.0 | | | | | |
| | (A) Ultraviolet absorbing compound (S-06) | | 4.0 | | | | |
| | (A) Ultraviolet absorbing compound (S-17) | | | 4.0 | | | |
| | (A) Ultraviolet absorbing compound (S-26) | | | | 4.0 | | |
| | (A) Ultraviolet absorbing compound (S-27) | | | | | 4.0 | |
| | (A) Ultraviolet absorbing compound (S-28) | | | | | | 4.0 |
| 8 | (B) Ultraviolet absorbing compound (V) | 4.0 | 4.0 | 4.0 | 4.0 | 4.0 | 4.0 |
| 9 | (B) Ultraviolet absorbing compound (IV) | 5.0 | 5.0 | 5.0 | 5.0 | 5.0 | 5.0 |
| 10 | Citric acid | 0.01 | 0.01 | 0.01 | 0.01 | 0.01 | 0.01 |
| 11 | Sodium citrate | 0.1 | 0.1 | 0.1 | 0.1 | 0.1 | 0.1 |
| 12 | Phenoxyethanol | 0.5 | 0.5 | 0.5 | 0.5 | 0.5 | 0.5 |
| 13 | Ethanol | 5.0 | 5.0 | 5.0 | 5.0 | 5.0 | 5.0 |
| 14 | Dynamite glycerin | 1.0 | 1.0 | 1.0 | 1.0 | 1.0 | 1.0 |
| 15 | Succinoglucan | 0.2 | 0.2 | 0.2 | 0.2 | 0.2 | 0.2 |
| 16 | Cellulose gum | 1.0 | 1.0 | 1.0 | 1.0 | 1.0 | 1.0 |
| 17 | Ion-exchanged water | bal. | bal. | bal. | bal. | bal. | bal. |

### Production Method

According to the prescription shown in Table 11, an ion-exchanged water solution (aqueous phase) containing the components 10 to 17 was prepared, and the solution was gradually added to the oil phase containing the components 1 to 9. Finally, the mixture was stirred using a homomixer (vacuum emulsification, Model PVQ-1D, manufactured by Mizuho Industrial Co., Ltd.).

### [Examples 7-25 to 7-30: Self-Tanning Cosmetic]

**Table 12**

| | Components | Amount Added (mass%) | | | | | |
|---|---|---|---|---|---|---|---|
| | | Example 7-25 | Example 7-26 | Example 7-27 | Example 7-28 | Example 7-29 | Example 7-30 |
| (I) | 1,3-Butylene glycol (produced by Kyowa Hakko Chemical) | 5.0 | 5.0 | 5.0 | 5.0 | 5.0 | 5.0 |
| | Glycerin | 2.0 | 2.0 | 2.0 | 2.0 | 2.0 | 2.0 |
| | Dihydroxyacetone | 5.0 | 5.0 | 5.0 | 5.0 | 5.0 | 5.0 |
| | Disodium edetate | 0.05 | 0.05 | 0.05 | 0.05 | 0.05 | 0.05 |
| | Paraben | q.s. | q.s. | q.s. | q.s, | q.s. | q.s. |
| | Ion-exchanged water | bal. | bal. | bal. | bal. | bal. | bal. |
| (II) | Glyceryl stearate (NIKKOL MGS-DEX) (produced by Nikko Chemicals) | 4.0 | 4.0 | 4.0 | 4.0 | 4.0 | 4.0 |
| | Behenyl alcohol (NIKKOL Behenyl Alcohol 65 (produced bey Nikko Chemicals) | 3.0 | 3.0 | 3.0 | 3.0 | 3.0 | 3.0 |
| | Stearyl alcohol (NIKKLOL Deodorant Stearyl Alcohol) (produced by Nikko Chemicals) | 2.0 | 2.0 | 2.0 | 2.0 | 2.0 | 2.0 |
| | Silicone oil | 6.0 | 6.0 | 6.0 | 6.0 | 6.0 | 6.0 |
| | Hydrogenated palm oil | 2.0 | 2.0 | 2.0 | 2.0 | 2.0 | 2.0 |
| | Liquid paraffin | 2.0 | 2.0 | 2.0 | 2.0 | 2.0 | 2.0 |
| | Zinc oxide surface-treated with octyltrimethoxysilane | 3.0 | 3.0 | 3.0 | 3.0 | 3.0 | 3.0 |
| | '(A) Ultraviolet absorbing compound (S-01) | 3.0 | | | | | |
| | (A) Ultraviolet absorbing compound (S-06) | | 3.0 | | | | |
| | (A) Ultraviolet absorbing compound (S-17) | | | 3.0 | | | |
| | (A) Ultraviolet absorbing compound (S-26) | | | | 3.0 | | |
| | (A) Ultraviolet absorbing compound (S-27) | | | | | 3.0 | |
| | (A) Ultraviolet absorbing compound (S-28) | | | | | | 3.0 |
| | (B) Ultraviolet absorbing compound (V) | 4.0 | 4.0 | 4.0 | 4.0 | 4.0 | 4.0 |
| | Perfume | q.s. | q.s. | q.s. | q.s. | q.s. | q.s. |

### Production Method

According to the prescription shown in Table 12, disodium edetate, dihydroxyacetone, glycerin and paraben dissolved under heating in 1,3-butylene glycol were added and dissolved in ion-exchanged water of (I). Also, respective components of (II) were mixed and thoroughly dissolved under heating at 80°C, and the resulting solution was added to (I). This mixture was emulsified and then cooled to room temperature to obtain a self-tanning cream.

### [Example 8]

### [Examples 8-1 to 8-6: Preparation of Sunscreen Cream]

The W/O sunscreen creams shown in Table 13 were prepared by the following method.

The oil-soluble components all were heated at 85°C in a vessel under stirring and when all components were dissolved or turned into a liquid state, the aqueous phase was mixed by homogenization. The obtained emulsion was cooled to about 40°C with stirring, thereby being homogenized, and further cooled to 25°C while continuing the stirring to obtain a sunscreen cream.

**Table 13**

| | Additive amount (mass%) | | | | | |
|---|---|---|---|---|---|---|
| Components | Example 8-1 | Example 8-2 | Example 8-3 | Example 8-4 | Example 8-5 | Example 8-6 |
| Mixture of glycerol mono/distearate and polyethylene glycol stearate 100 OE (ARALACEL 165 FL-ICI) | 1.0 | 1.0 | 1.0 | 1.0 | 1.0 | 1.0 |
| Cetanol (NIKKOL Deodorant Cetanol 70) (produced by Nikko Chemicals) | 0.5 | 0.5 | 0.5 | 0.5 | 0.5 | 0.5 |
| Palm oil stearic acid (STEARINE TP-STEARINERIE DUBOIS) | 2.5 | 2.5 | 2.5 | 2.5 | 2.5 | 2.5 |
| Polydimethylsiloxane (DOW CORNING 200 FLUID-DOW CORNING) | 0.5 | 0.5 | 0.5 | 0.5 | 0.5 | 0.5 |
| Benzoate of C12/C15 alcohol (WITCONOL TN-WITCO) (produced by WITCO) | 20.0 | 20.0 | 20.0 | 20.0 | 20.0 | 20.0 |
| (A) Ultraviolet absorbing compound (S-01) | 2.0 | | | | | |
| (A) Ultraviolet absorbing compound (S-06) | | 2.0 | | | | |
| (A) Ultraviolet absorbing compound (S-17) | | | 2.0 | | | |
| (A) Ultraviolet absorbing compound (S-26) | | | | 2.0 | | |
| (A) Ultraviolet absorbing compound (S-27) | | | | | 2.0 | |
| (A) Ultraviolet absorbing compound (S-28) | | | | | | 2.0 |
| (B) Ultraviolet absorbing compound (V) | 1.0 | 1.0 | 1.0 | 1.0 | 1.0 | 1.0 |
| Glycerin | 5.0 | 5.0 | 5.0 | 5.0 | 5.0 | 5.0 |
| Hexadecyl alcohol phosphate, sodium salt (AMPHISOL K-HOFFMANN LA ROCHE) (produced by F. *Hoffmann-La Roche)* | 1.0 | 1.0 | 1.0 | 1.0 | 1.0 | 1.0 |
| Polyacrylic acid (SYNTHALEN K-3V) | 0.3 | 0.3 | 0.3 | 0.3 | 0.3 | 0.3 |
| Hydroxypropylmethylcellulose (METHOCEL F4M-DOW CHEMICAL) | 0.1 | 0.1 | 0.1 | 0.1 | 0.1 | 0.1 |
| Cyclopentadimethylsiloxane (DC245-DOW CORNING) | 2 | 2 | 2 | 2 | 2 | 2 |
| Triethanolamine | 0.8 | 0.8 | 0.8 | 0.8 | 0.8 | 0.8 |
| Preservative | q.s. | q.s. | q.s. | q.s. | q.s. | q.s. |
| Deionized water | bal. | bal. | bal. | bal. | bal. | bal. |

### [Example 9]

### [Examples 9-1 to 9-6: Preparation of Sunscreen Cream]

The W/O sunscreen creams shown in Table 14 were prepared by the following method.

The oil-soluble components all were heated at 85°C in a vessel under stirring and when all components were dissolved or turned into a liquid state, the aqueous phase was mixed by homogenization. The obtained emulsion was cooled to about 40°C with stirring, thereby being homogenized, and further cooled to 25°C while continuing the stirring to obtain a sunscreen cream.

**Table 14**

| Components | Amount Added (mass%) | | | | | |
|---|---|---|---|---|---|---|
| | Example 9-1 | Example 9-2 | Example 9-3 | Example 9-4 | Example 9-5 | Example 9-6 |
| Mixture of cetylstearyl alcohol and oxyethylenated cetylstearyl alcohol, 33OE (80/20) (SINNOWAX AO-HENKEL) | 7.0 | 7.0 | 7.0 | 7.0 | 7.0 | 7.0 |
| Mixture of glyceryl mono- and distearate (CERASYNT SD-V produced by ISP) | 2.0 | 2.0 | 2.0 | 2.0 | 2.0 | 2.0 |
| Cetanol (NIKKOL Deodorant Cetanol 70) (produced by Nikko Chemicals) | 1.5 | 1.5 | 1.5 | 1.5 | 1.5 | 1.5 |
| Polydimethylsiloxane (produced by Dow Coming Toray) (DOW CORNING 200 FLUID-DOW CORNING) | 1.5 | 1.5 | 1.5 | 1.5 | 1.5 | 1.5 |
| Benzoate of C12/C15 alcohol (produced by WITCO) (WITCONOL TN-WITCO) | 8.0 | 8.0 | 8.0 | 8.0 | 8.0 | 8.0 |
| Vaseline oil | 10.0. | 10.0. | 10.0. | 10.0. | 10.0. | 10.0. |
| (A) Ultraviolet absorbing compound (S-01) | 2.0 | | | | | |
| (A) Ultraviolet absorbing compound (S-06) | | 2.0 | | | | |
| (A) Ultraviolet absorbing compound (S-17) | | | 2.0 | | | |
| (A) Ultraviolet absorbing compound (S-26) | | | | 2.0 | | |
| (A) Ultraviolet absorbing compound (S-27) | | | | | 2.0 | |
| (A) Ultraviolet absorbing compound (S-28) | | | | | | 2.0 |
| (B) Ultraviolet absorbing compound (V) | 1 | 1 | 1 | 1 | 1 | 1 |
| Glycerin | 10 | 10 | 10 | 10 | 10 | 10 |
| Preservative | q.s. | q.s. | q.s. | q.s. | q.s. | q.s. |
| Deionized water | bal. | bal. | bal. | bal. | bal. | bal. |

### [Example 10]

### [Examples 10-1 to 10-6, Comparative Examples 10 and 11: Preparation of Sunscreen Cream]

The sunscreen creams shown in Table 15 were prepared as follows.

### (Production Method)

Propylene glycol and ultraviolet absorbing compounds (A) and (B) were added to the ion-exchanged water and dissolved, and the resulting solution was heated and kept at 70°C (aqueous phase). Other components were mixed, and the mixture was melted under heating and kept at 70°C (oil phase). The oil phase was added to the aqueous phase, and the resulting mixture was subjected to pre-emulsification, further uniformly emulsified by a homomixer and then cooled 30°C while thoroughly stirring it.

The results are shown in Table 16.

**Table 15**

| Component | Amount Added (mass%) |
|---|---|
| Stearyl alcohol (NIKKOL Deodorant Stearyl Alcohol) (produced by Nikko Chemicals) | 7.0 |
| Stearic acid | 2.0 |
| Lanolin (PEG-20 Lanolin) (produced by Nikko Chemicals) | 2.0 |
| Squalane (NIKKOL Squalane) (produced by Nikko Chemicals) | 5.0 |
| 2-Octyldodecyl alcohol | 6.0 |
| Polyethylene glycol monocetyl ether | 3.0 |
| Glyceryl stearate (NIKKOL MGS-DEX) (produced by Nikko Chemicals) | 2.0 |
| Propylene glycol | 5.0 |
| (A) Ultraviolet absorbing compound (shown in Table 16) | 5 to 15 |
| (B) Ultraviolet absorbing compound (II) | 4.0 |
| (B) Ultraviolet absorbing compound (V) | 4.0 |
| Perfume | q.s. |
| Sodium bisulfite | 0.03 |
| Ethyl paraben | 0.3 |
| Ion-exchanged water | bal. |

**Table 16**

| | (A) Ultraviolet Absorbing Compound | Blending Amount | Appearance | Precipitation property |
|---|---|---|---|---|
| Example 10-1 | S-01 | 15 | colorless and transparent | not precipitated |
| | | 10 | colorless and transparent | not precipitated |
| | | 5 | colorless and transparent | not precipitated |
| Example 10-2 | S-06 | 15 | colorless and transparent | not precipitated |
| | | 10 | colorless and transparent | not precipitated |
| | | 5 | colorless and transparent | not precipitated |
| Example 10-3 | S-17 | 15 | colorless and transparent | not precipitated |
| | | 10 | colorless and transparent | not precipitated |
| | | 5 | colorless and transparent | not precipitated |
| Example 10-4 | S-26 | 15 | colorless and transparent | not precipitated |
| | | 10 | colorless and transparent | not precipitated |
| | | 5 | colorless and transparent | not precipitated |
| Example 10-5 | S-27 | 15 | colorless and transparent | not precipitated |
| | | 10 | colorless and transparent | not precipitated |
| | | 5 | colorless and transparent | not precipitated |
| Example 10-6 | S-28 | 15 | colorless and transparent | not precipitated |
| | | 10 | colorless and transparent | not precipitated |
| | | 5 | colorless and transparent | not precipitated |
| Comparative Example 10 | Tinovin 460 produced by Ciba (CAS No. 208343-47-9) | 15 | yellow and transparent | precipitated |
| | | 10 | yellow and transparent | not precipitated |
| | | 5 | colorless and transparent | not precipitated |
| Comparative Example 11 | - | no blending | colorless and transparent | - |

The entire disclosure of Japanese Patent Application No. 2009-206477 filed on September 7, 2009, from which the benefit of foreign priority has been claimed in the present application, and the entire disclosure of Japanese Patent Application No. 2010-195218 filed on August 31, 2010, are incorporated herein by reference, as if fully set forth.

## Claims

1. An ultraviolet absorbing composition for skin and hair, which comprises an ultraviolet absorbing compound represented by formula (I) and at least one ultraviolet absorbing compound selected from the group consisting of formulae (I) to (V): 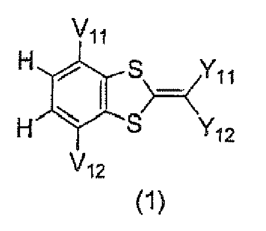 In the formula (I), Y₁₁ and Y₁₂ each independently represents a monovalent substituent; one of Y₁₁ and Y₁₂ is cyano group, and the other is cyano group, substituted or unsubstituted alkylcarbonyl group, substituted or unsubstituted arylcarbonyl group, substituted or unsubstituted heterocyclic carbonyl group, substituted or unsubstituted alkylsulfonyl group, substituted or unsubstituted arylsulfonyl group, substituted or unsubstituted carbamoyl group, substituted or unsubstituted sulfamoyl group, substituted or unsubstituted alkoxycarbonyl group, and substituted or unsubstituted aryloxycarbonyl group; and V₁₁ and V₁₂ each independently represents a hydrogen atom or a monovalent substituent; 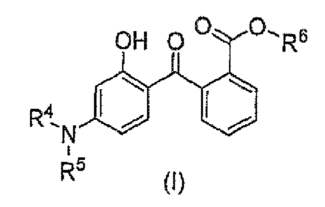 In formula (I), R⁴ to R⁶ each independently represents a hydrogen atom, an alkyl group or a cycloalkyl group; R⁴ and R⁵ can be linked each other to form a 5-membered ring or 6-membered ring.

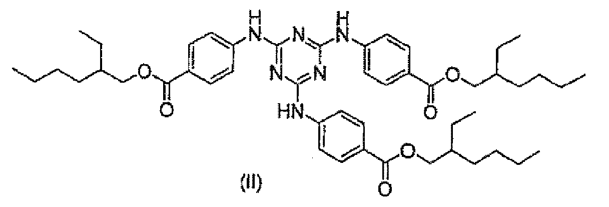

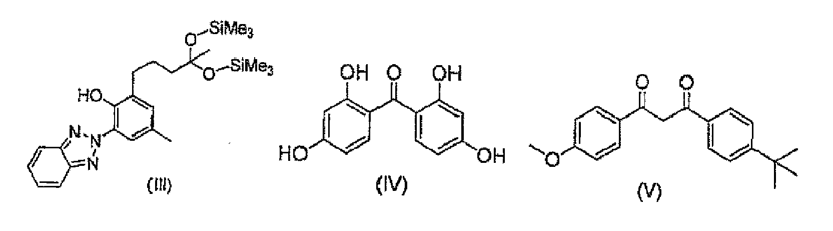

2. The ultraviolet absorbing composition for skin and hair according to claim 1, wherein the ultraviolet absorbing compound represented by formula (1) is an ultraviolet absorbing compound represented by formula (2): 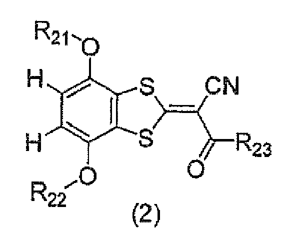 wherein R₂₁ and R₂₂ each independently represents unsubstituted alkyl group or unsubstituted alkylcarbonyl group; and R₂₃ represents unsubstituted alkyl group or unsubstituted aryl group.

3. The ultraviolet absorbing composition for skin and hair according to claim 1 or 2, wherein at least one ultraviolet absorbing compound selected from the group consisting of formulae (I) to (V) is a compound represented by formula (V).
